# EUROPEAN PATENT SPECIFICATION

(11) **EP 1 290 137 B1**
(45) Date of publication and mention of the grant of the patent: **21.02.2007**
(21) Application number: 01931888.0
(22) Date of filing: 18.05.2001
(51) Int. Cl.: C12N 1/36, C12N 15/31, C07K 14/22, C07K 16/12, A61K 39/095, A61P 37/02

(54) **VIRULENCE GENES, PROTEINS, AND THEIR USE**
VIRULENZ GENE, PROTEINE, UND IHRE VERWENDUNG
GENES DE VIRULENCE, PROTEINES, ET LEUR UTILISATION

(30) Priority: 18.05.2000 GB 0012079
(43) Date of publication of application: 12.03.2003
(73) Proprietor: Microscience Limited, Wokingham, Berkshire RG41 5TU (GB)
(72) Inventor: TANG, Christoph Centre for Molecular Micr. & Inf., Armstrong Road London SW7 2AZ (GB)
(74) Representative: Jappy, John William Graham
(86) International application number: PCT/GB2001/002247
(87) International publication number: WO 2001/087939

(56) References cited:
- WO-A-98/56901
- LLOYD R G ET AL: "MOLECULAR ORGANIZATION AND NUCLEOTIDE SEQUENCE OF THE RECG LOCUS OF ESCHERICHIA-COLI K-12" JOURNAL OF BACTERIOLOGY, vol. 173, no. 21, 1991, pages 6837-6843, XP001013416 ISSN: 0021-9193
- DATABASE EMBL [Online] retrieved from EBI Database accession no. NMA2Z2491 XP002175426
- DATABASE EMBL [Online] retrieved from EBI Database accession no. AE002528 XP002175427
- SUN Y ET AL: "Identification of genes required for competence in Neisseria meningitidis." ABSTRACTS OF THE GENERAL MEETING OF THE AMERICAN SOCIETY FOR, vol. 100, 2000, page 270 XP001012784 100th General Meeting of the American Society for Microbiology;Los Angeles, California, USA; May 21-25, 2000, 2000 ISSN: 1060-2011
- TANG C ET AL: "For discussion: live attenuated vaccines for group B meningococcus" VACCINE, BUTTERWORTH SCIENTIFIC. GUILDFORD, GB, vol. 17, no. 2, 1999, pages 114-117, XP004139965 ISSN: 0264-410X
- MORRISON D A ET AL: "ISOLATION OF TRANSFORMATION-DEFICIENT STREPTOCOCCUS PNEUMONIAE MUTANTS DEFECTIVE IN CONTROL OF COMPETENCE, USING INSERTION-DUPLICATION MUTAGENESIS WITH THE ERYTHROMYCIN RESISTANCE DETERMINANT OF PAMBETA1" JOURNAL OF BACTERIOLOGY, WASHINGTON, DC, US, vol. 159, no. 3, 1 September 1984 (1984-09-01), pages 870-876, XP000650381 ISSN: 0021-9193
- MARTIN B ET AL: "THE MMSA LOCUS OF STREPTOCOCCUS PNEUMONIAE ENCODES A RECG-LIKE PROTEIN INVOLVED IN DNA REPAIR AND IN THREE-STRAND RECOMBINATION" MOLECULAR MICROBIOLOGY, BLACKWELL SCIENTIFIC, OXFORD, GB, vol. 19, no. 5, March 1996 (1996-03), pages 1035-1045, XP001008415 ISSN: 0950-382X
- MORTIER-BARRIERE ISABELLE ET AL: "Control of recombination rate during transformation of Streptococcus pneumoniae: An overview." MICROBIAL DRUG RESISTANCE, vol. 3, no. 3, 1997, pages 233-242, XP001013410 ISSN: 1076-6294
- CLAUS H ET AL: "Identification of a hotspot for transformation of Neisseria meningitidis by shuttle mutagenesis using signature-tagged transposons." MOLECULAR AND GENERAL GENETICS, vol. 259, no. 4, 1998, pages 363-371, XP002175425 ISSN: 0026-8925
- JYSSUM: "Genetic Factors determining competence in transformation of Neisseria meningitidis" ACTA PATH ET MICROBIOL SCANDINAV, vol. 67, 1966, pages 493-502, XP001013546
- FUSSENEGGER M ET AL: "Transformation competence and type-4 pilus biogenesis in Neisseriagonorrhoeae - areview" GENE: AN INTERNATIONAL JOURNAL ON GENES AND GENOMES, ELSEVIER SCIENCE PUBLISHERS, BARKING, GB, vol. 192, no. 1, 11 June 1997 (1997-06-11), pages 125-134, XP004115696 ISSN: 0378-1119
- HOLMES L ET AL: "In vivo evaluation of live attenuated Neisseria meningitidis serogroup B constructs." IMMUNOLOGY, vol. 101, no. Supplement 1, December 2000 (2000-12), page 34 XP001013651 Annual Congress of the British Society for Immunology;Harrogate, UK; December 05-08, 2000 ISSN: 0019-2805

## Description

### Field of the Invention

This invention relates to bacterial genes and proteins, and their use. More particularly, it relates to genes and proteins/peptides obtained from *Neisseria meningitidis,* and their use in therapy and in screening for drugs.

### Background of the Invention

*Neisseria* meningitidis is a Gram-negative bacterial pathogen that is implicated in septic shock and bacterial meningitis. This bacterium is a leading cause of bacterial meningitis in developed countries, and causes large-scale epidemics in Africa and China. In the UK, *Neisseria meningitidis* is the leading cause of death in childhood apart from road traffic accidents. The bacterium naturally inhabits the human naso-pharynx and then gains access to the blood stream from where it causes severe septicaemia or meningitis. Although current anti-microbials are effective in eliminating the bacterium from the body, the mortalilty from menigococcal septicaemia remains substantial.

Immunisation with avirulent N. *meningitidis* microorganisms has also been proposed. Avirulence is achieved through genetic modification to disrupt the expression of genes implicated in virulence. One concern is that a mutant strain may revert to its virulent form by taking up replacement DNA. It is therefore desirable to provide means for treating or preventing conditions caused by *Neisseria meningitidis,* e.g. by immunisation.

### Summary of the Invention

The present invention is based on the discovery of genes in *Neisseria meningitidis* that are required for natural competence (uptake and incorporation of exogenous DNA).

The genes identified herein may be utilised to prepare microorganisms that are transformation-deficient. The mutant microorganisms will usually have a mutation that disrupts the expression of one or more of the genes identified herein, to provide a strain that lacks the ability to uptake DNA. These microorganisms will also have use in therapy and diagnosis.

According to a first aspect of the invention, a microorganism comprises a mutation that disrupts the expression of a gene comprising any of the nucleotide sequences identified in claim 1, or a homologue thereof with at least 40% sequence identity, for therapeutic or diagnostic use.

The microorganisms may have many therapeutic uses for treating *Neisseria* infections, including use in vaccines for prophylactic application.

According to a second aspect, a peptide is encoded by an operon including any of the nucleotide sequences identified in claim 1.

### Description of the Invention

The present invention is based on the discovery of genes encoding peptides which are responsible for the uptake of DNA. The peptides and genes of the invention are therefore useful for the preparation of therapeutic agents to treat infection. It should be understood that references to therapy also include preventative treatments, e.g. vaccination. Furthermore, while the products of the invention are intended primarily for treatment of infections in human patients, veterinary applications are also considered to be within the scope of the invention.

The present invention is described with reference to *Neisseria meningitidis.* However, all the *Neisseria* strains, and many other Gram-negative bacterial strains are likely to include related peptides or proteins having amino acid sequence identity or similarity to those identified herein. Organisms likely to contain the peptides include, but are not limited to the genera *Salmonella, Enterobacter, Klebsiella, Shigella* and *Yersinia.*

Preferably, the peptides that may be useful in the various aspects of the invention have greater than a 40% similarity with the peptides identified herein. More preferably, the peptides have greater than 60% sequence similarity. Most preferably, the peptides have greater than 80% sequence similarity, e.g. 95% similarity. With regard to the polynucleotide sequences identified herein, related polynucleotides that may be useful in the various aspects of the invention may have greater than 40% identity with the sequences identified herein. More preferably, the polynucleotide sequences have greater than 60% sequence identity. Most preferably, the polynucleotide sequences have greater than 80% sequence identity, e.g. 95% identity.

The terms "similarity" and "identity" are known in the art. The use of the term "identity" refers to a sequence comparison based on identical matches between correspondingly identical positions in the sequences being compared. The term "similarity" refers to a comparison between amino acid sequences, and takes into account not only identical amino acids in corresponding positions, but also functionally similar amino acids in corresponding positions. Thus similarity between polypeptide sequences indicates functional similarity, in addition to sequence similarity.

Levels of identity between gene sequences and levels of identity or similarity between amino acid sequences can be calculated using known methods. In relation to the present invention, publicly available computer based methods for determining identity and similarity include the BLASTP, BLASTN and FASTA (Atschul *et al*., J. Molec. Biol., 1990; 215:403-410), the BLASTX program available from NCBI, and the Gap program from Genetics Computer Group, Madison WI. The levels of similarity and identity provided herein, were obtained using the Gap program, with a Gap penalty of 12 and a Gap length penalty of 4 for determining the amino acid sequence comparisons, and a Gap penalty of 50 and a Gap length penalty of 3 for the polynucleotide sequence comparisons.

Having characterised a gene according to the invention, it is possible to use the gene sequence to search for related genes or peptides in other microorganisms. This may be carried out by searching in existing databases, e.g. EMBL or GeneBank.

Peptides or proteins according to the invention may be purified and isolated by methods known in the art. In particular, having identified the gene sequence, it will be possible to use recombinant techniques to express the genes in a suitable host. Active fragments and related molecules can be identified and may be useful in therapy. For example, the peptides or their active fragments may be used as antigenic determinants in a vaccine, to elicit an immune response. They may also be used in the preparation of antibodies, for passive immunisation, or diagnostic applications. Suitable antibodies include monoclonal antibodies, or fragments thereof, including single chain Fv fragments. Methods for the preparation of antibodies will be apparent to those skilled in the art.

Active fragments of the peptides are those that retain the biological function of the peptide, i.e. retain the ability to take up DNA. Typically, the fragment will be at least 30 nucleotides (10 amino acids) in size, preferably 60 nucleotides (20 amino acids) and most preferably greater than 90 nucleotides (30 amino acids) in size.

The genes identified herein are likely to be useful both in generating vaccine strains that cannot take up exogenous DNA, and as a target for antimicrobials. Attenuated microorganisms can be produced with an attenuating mutation in a virulence gene or genes together with a mutation that disrupts a gene or genes of the invention, providing an attenuated microorganism that cannot take up extracellular DNA.

The term "virulence" is known in the art and refers to the pathogenicity of a microorganism. The use of the term "avirulent" is intended therefore to refer to microorganisms that are no longer pathogenic.

Suitable virulence genes will be apparent to the skilled person, and include auxotroph genes and those identified in Sun *et al.,* Nature medicine, 2000; 6(11): 1269-1273.

The skilled person will be aware of methods for disrupting the expression of particular genes. Techniques that may be used include insertional inactivation or gene deletion techniques. Attenuated microorganisms according to the invention may also comprise additional mutations in other genes.

The preparation of vaccines based on attenuated microorganisms is known to those skilled in the art. Vaccine compositions can be formulated with suitable carriers or adjuvants, e.g. alum, as necessary or desired, to provide effective immunisation against infection. The preparation of vaccine formulations will be apparent to the skilled person.

Attenuated microorganisms may also be used as carrier systems for the delivery of heterologous antigens, therapeutic proteins or nucleic acids (DNA or RNA). In this embodiment, the attenuated microorganisms are used to deliver a heterologous antigen, protein or nucleic acid to a particular site in vivo. Introduction of a heterologous antigen, peptide or nucleic acid into an attenuated microorganism can be carried out by conventional techniques, including the use of recombinant constructs, e.g. vectors, which comprise polynucleotides that express the heterologous antigen or therapeutic protein, and also include suitable promoter sequences. As the microorganisms are transformation-deficient, it will be necessary to incorporate heterologous nucleic acids via an active mechanism, e.g. electroporation. This is a known technique. Alternatively, the gene that encodes the heterologous antigen or protein may be incorporated into the genome of the organism and the endogenous promoters used to control expression. A further alternative is to modify the microorganisms to disrupt the genes identified herein after first transforming with the heterologous gene.

More generally, and as is well known to those skilled in the art, a suitable amount of an active component of the invention can be selected, for therapeutic use, as can suitable carriers or excipients, and routes of administration. These factors would be chosen or determined according to known criteria such as the nature/severity of the condition to be treated, the type and/or health of the subject etc.

The various products of the invention may also be used in veterinary applications.

The following is a brief overview of the experimental procedure used to identify the genes.

Signature-tagged mutagenesis (STM) (Hensel *et al.,* Science, 1995; 269: 400-403) was used to identify the genes in *N. meningitidis* that are essential for uptake of DNA. In STM, individual mutants are tagged with unique sequence identifiers, allowing large numbers of mutants to be analysed simultaneously.

A *Neisseria meningitidis* serogroup B Tn10 mutant bank was used to screen for mutants that exhibited a transformation defect. Pools of mutants were transformed with either plasmid or chromosomal DNA carrying an erythromycin-resistance marker. Transformation-deficient mutants were identified by plating out transformed pools of mutant bacteria onto solid media, in the presence and absence of erythromycin and performing a STM screen. Bacteria containing a modified version of the transposon Tn10 inserted within a competence gene failed to be recovered from plates containing erythromycin inoculated with a mixed population of mutants, and were, therefore likely to be defective in the uptake of exogenous DNA.

The cloned *Neisseria meningi tidis* nucleotide sequences immediately following the Tn10 insertion were identified, and their translated sequences characterised. Homologues were then searched for in existing protein and DNA databases (www.sanger.ac.uk/Projects/N-meningitidis; www.tigr.org; and www.ncbi.nlm.nih.gov).

The selected mutants were tested for the ability to take up exogenous DNA and were shown to take up exogenous DNA at levels at least 5 fold below wild-type levels. As the genes are transcribed as part of an operon it is possible that the mutation is due to a polar effect on a downstream gene.

To prevent the identification of mutants that merely could not express an erythromycin resistant or capsule-minus phenotype, all putatively competence-defective mutants were also analysed for their ability to be transformed to tetracycline resistance. The results confirmed that the mutants were transformation deficient.

The genes of the invention are shown in Table 1 together with the identification of any known homologue. In addition, Table 1 shows the transformation frequency, illustrating the deficiency of DNA uptake exhibited by the mutants, compared to wild-type.

**Table 1**

| | | | | |
|---|---|---|---|---|
| **SEQ ID NO.** | **Genes** | **Function** | **Transformation frequency** | **Accession number** |
| 1 | *rec*G | ATP-dependent DNA helicase RECG | 1/5 | NMB1788 |
| 3 | *rho* | Transcription termination factor RHO | 0 | NMB0617 |
| 5 | *pil*N | PBP1 | 0 | NMB1809 |
| 7 | *phl*l | Phosphoenolpyruvate-protein phosphotransferase | 1/30 | NMB2044 |
| 9 | *cut*E | *E*. *coli* apolipoprotein N-acyltransferase | 1/10 | NMB0713 |
| 11 | *smf* | SMF protein (DNA processing chain A) | 0 | NMB0116 |
| 13 | | CcsA-related protein | 1/2000 | NMB0296 |
| 15 | *vac*B | *E*. *coli* Vacb protein | 1/10 | NMB1200 |
| 17 | | No hit in NM nucleic acid database | 1/5 | |
| 19 | *pro*C | Pyrroline-5-carboxylate reductase | 0 | NMB055 |
| 21 | | Phosphoribosylglycinamide formyltransferase | 1/40 | NMB1566 |

## Claims

1. A transformation - deficient microorganism comprising a first mutation that disrupts the expression of a first gene comprising the nucleotide sequence identified herein as SEQ ID NO. 1, or a related molecule having at least 60% sequence identity, for therapeutic use.

2. A microorganism according to claim 1, further comprising a second mutation that disrupts the expression of a virulence gene.

3. A microorganism according to claim 1 or claim 2, wherein the first mutation results in insertional inactivation or a gene deletion.

4. A microorganism according to any preceding claim, wherein the microorganism is *Neisseria meningitidis.*

5. A microorganism according to any preceding claim, comprising a heterologous antigen, therapeutic peptide or nucleic acid.

6. A microorganism according to any preceding claim, wherein the sequence identity is at least 90%.

7. A microorganism according to any of claims 1 to 5, wherein the first gene normally encodes a peptide comprising the amino acid sequence identified herein as SEQ ID NO. 2.

8. A vaccine comprising a microorganism according to any preceding claim.

9. Use of a product according to any of claims 1 to 8 for the manufacture of a medicament for use in the treatment or prevention of a condition associated with infection by *Neisseria* or Gram-negative bacteria.

10. Use according to claim 9, wherein the condition is meningitis.

11. Use according to claim 9 or claim 10, for veterinary treatment.

## Patentansprüche

1. Transformationsdefizienter Mikroorganismus zur therapeutischen Verwendung, der eine erste Mutation umfasst, welche die Expression eines ersten Gens, umfassend die vorliegend als SEO ID NO.1 definierte Nukleotidsequenz, oder eines verwandten Moleküls mit einer Sequenzidentität von mindestens 60% aufhebt.

2. Mikroorganismus nach Anspruch 1, der außerdem eine zweite Mutation umfasst, welche die Expression eines Virulenzgens aufhebt.

3. Mikroorganismus bei Anspruch 1 oder Anspruch 2, wobei die erste Mutation in einer Insertionsinaktivierung oder einer Gendeletion resultiert.

4. Mikroorganismus nach einem vorhergehenden Anspruch, wobei der Mikroorganismus *Neisseria meningitidis* ist.

5. Mikroorganismus nach einem vorhergehenden Anspruch, der ein heterologes Antigen, therapeutisches Peptid oder Nukleinsäure umfasst.

6. Mikroorganismus nach einem vorhergehenden Anspruch, wobei die Sequenzidentität mindestens 90% beträgt.

7. Mikroorganismus nach einem der Ansprüche 1 bis 5, wobei das erste Gen normalerweise für ein Peptid kodiert, das die vorliegend als SEO ID NO. 2 definierte Aminosäuresequenz umfasst.

8. Vakzin, das einen Mikroorganismus nach einem vorhergehenden Anspruch umfasst.

9. Verwendung eines Produkts nach einem der Ansprüche 1 bis 8 zur Herstellung eines Medikaments zur Verwendung in der Behandlung oder Prävention eines Zustands, der mit einer Infektion durch *Neisseria* oder Gram - negative Bakterien assoziert ist.

10. Verwendung nach Anspruch 9, wobei der Zustand eine Meningitis ist.

11. Verwendung nach Anspruch 9 oder Anspruch 10 zur tiermedizinischen Behandlung.

## Revendications

1. Microorganisme à transformation déficiente comprenant une première mutation qui interrompt l'expression d'un premier gène comprenant la séquence nucléotide identifiée ici comme SEQ ID NO.1, ou une molécule apparentée ayant au moins 60% d'identité de séquence, pour usage thérapeutique.

2. Microorganisme selon la revendication 1, comprenant de plus une seconde mutation qui interrompt l'expression d'un gène de virulence.

3. Microorganisme selon la revendication 1 ou la revendication 2, dans lequel la première mutation se traduit par une inactivation par insertion ou une délétion de gène.

4. Microorganisme selon l'une quelconque des revendications précédentes, dans lequel le microorganisme est le *Neisseria meningiditis.*

5. Microorganisme selon l'une quelconque des revendications précédentes, comprenant un antigène hétérologue, un peptide thérapeutique ou un acide nucléique.

6. Microorganisme selon l'une quelconque des revendications précédentes, dans lequel l'identité de séquence est au moins de 90 %.

7. Microorganisme selon l'une quelconque des revendications 1 à 5, dans lequel le premier gène code normalement un peptide comprenant la séquence d'acides aminés identifiée ici comme étant SEQ ID NO.2.

8. Vaccin comprenant un microorganisme selon l'une quelconque des revendications précédentes.

9. Utilisation d'un produit selon l'une quelconque des revendications 1 à 8 pour la fabrication d'un médicament destiné au traitement ou à la prévention d'une condition associée à l'infection par une *Neisseria* ou une bactérie Gram-négatif.

10. Utilisation selon la revendication 9, dans lequel la condition est la méningite.

11. Utilisation selon la revendication 9 ou la revendication 10 pour un traitement vétérinaire.


### SEQUENCE LISTING

<110> Microscience Limited
<120> Virulence Genes, Proteins, and Their Use
<130> REP06443WO
<140> (not yet known)
   <141> 2001-05-18
<150> 0012079.0
   <151> 2000-05-18
<160> 22
<170> PatentIn Ver. 2.1
<210> 1
   <211> 2040
   <212> DNA
   <213> Neisseria meningitidis
<220>
   <221> CDS
   <222> (1)..(2040)
<400> 1
   atg atg tcg ccc gaa acc cga aaa cag ctc aaa atc acc gat gtt tcc    48
   Met Met Ser Pro Glu Thr Arg Lys Gln Leu Lys Ile Thr Asp Val Ser
     1               5                  10                  15
   gcc aag aag ctc gac aaa ctc aac ctc cat acc gcg tgg gat ttg gtg    96
   Ala Lys Lys Leu Asp Lys Leu Asn Leu His Thr Ala Trp Asp Leu Val
                20                  25                  30
   ttg cac ctg ccg ctg cgt tac gag gac gag acg cac att atg ccg att   144
   Leu His Leu Pro Leu Arg Tyr Glu Asp Glu Thr His Ile Met Pro Ile
            35                  40                  45
   aag gac gcg ccg att ggc gtg ccg tgt cag gtc gag ggg gag gtt atc   192
   Lys Asp Ala Pro Ile Gly Val Pro Cys Gln Val Glu Gly Glu Val Ile
        50                  55                  60
   cat cag gaa gta acg ttc aaa ccg cgc aag cag ctg att gtc caa atc   240
   His Gln Glu Val Thr Phe Lys Pro Arg Lys Gln Leu Ile Val Gln Ile
    65                  70                  75                  80
   gcc gac ggc tcc ggc agc gtc ctt ttt ctg cgc ttc atc cac ttt tac   288
   Ala Asp Gly Ser Gly Ser Val Leu Phe Leu Arg Phe Ile His Phe Tyr
                       85                  90                  95
   gcc agc cat cag aaa cag acg gcg atc ggc aaa cgc atc cgc gcc gtg   336
   Ala Ser His Gln Lys Gln Thr Ala Ile Gly Lys Arg Ile Arg Ala Val
               100                 105                 110
   ggc gaa atc aaa cac ggc ttt tac ggc gac gag atg att cat ccc aaa   384
   Gly Glu Ile Lys His Gly Phe Tyr Gly Asp Glu Met Ile His Pro Lys
           115                 120                 125
   atc cgc gat gcc gag ggc ggc ggt ttg gcg gaa agc ctc acg ccg gtt   432
   Ile Arg Asp Ala Glu Gly Gly Gly Leu Ala Glu Ser Leu Thr Pro Val
       130                 135                 140
   tac ccg acc gta aac ggt ttg aac cag ccc act ttg cgc cgt att att   480
   Tyr Pro Thr Val Asn Gly Leu Asn Gln Pro Thr Leu Arg Arg Ile Ile
   145                 150                 155                 160
   cag acg gcg ttg gac gtt acg ccg ctg cac gac acg ctg ccc gat gcc   528
   Gln Thr Ala Leu Asp Val Thr Pro Leu His Asp Thr Leu Pro Asp Ala
                   165                 170                 175
   ctg ctg tgc cgt ctg aag ctg cca cac ctc gcc gaa agc ctg cgt ctt   576
   Leu Leu Cys Arg Leu Lys Leu Pro His Leu Ala Glu Ser Leu Arg Leu
               180                 185                 190
   ttg cat tcg ccg ccg ccg agt ttc acg att cat cag ctt tca gac ggc   624
   Leu His Ser Pro Pro Pro Ser Phe Thr Ile His Gln Leu Ser Asp Gly
           195                 200                 205
   acg ctg cct gca tgg caa cgg ctc aaa ttc gac gaa ctt ttg gcg caa   672
   Thr Leu Pro Ala Trp Gln Arg Leu Lys Phe Asp Glu Leu Leu Ala Gln
       210                 215                 220
   cag ctt tcg atg cgc ttg gcg cga cag aag cgt atc ggc ggc acg gcg   720
   Gln Leu Ser Met Arg Leu Ala Arg Gln Lys Arg Ile Gly Gly Thr Ala
   225                 230                 235                 240
   gcg gca ttg ggc ggc gac ggc aca ttg acc caa gcc ctg cgc caa gcc   768
   Ala Ala Leu Gly Gly Asp Gly Thr Leu Thr Gln Ala Leu Arg Gln Ala
                   245                 250                 255
   ctg ccg ttt gcc ctg acc gat gcg caa gaa aaa gtt gtt tcc gaa atc   816
   Leu Pro Phe Ala Leu Thr Asp Ala Gln Glu Lys Val Val Ser Glu Ile
               260                 265                 270
   tgc cgc gat atg gcg caa acc tac ccg atg cac cgc ctg ctg cag ggc     864
   Cys Arg Asp Met Ala Gln Thr Tyr Pro Met His Arg Leu Leu Gln Gly
           275                 280                 285
   gat gtc ggc agc ggc aaa acc att gtg gct gct ttg tct gct ttg acg     912
   Asp Val Gly Ser Gly Lys Thr Ile Val Ala Ala Leu Ser Ala Leu Thr
       290                 295                 300
   gct atc gaa tcc ggt gcg cag gtg gct gta atg gcg ccc act gaa atc     960
   Ala Ile Glu Ser Gly Ala Gln Val Ala Val Met Ala Pro Thr Glu Ile
   305                 310                 315                 320
   ctt gcc gaa cag cat ttt att aaa ttt aaa caa tgg ctc gaa cct ttg     1008
   Leu Ala Glu Gln His Phe Ile Lys Phe Lys Gln Trp Leu Glu Pro Leu
                   325                 330                 335
   ggc att gaa gtt gtc cgc ctt ttt ggc agt ttg cgt aaa aaa gcc aaa     1056
   Gly Ile Glu Val Val Arg Leu Phe Gly Ser Leu Arg Lys Lys Ala Lys
               340                 345                 350
   gac gaa gcc aaa gcc aaa ctg gct gac ggc agc gtc aaa atc gcc gtc     1104
   Asp Glu Ala Lys Ala Lys Leu Ala Asp Gly Ser Val Lys Ile Ala Val
           355                 360                 365
   ggc acg cac gcc ctg ttt tca gac ggc gtg gcg ttt cac aat ttg ggc     1152
   Gly Thr His Ala Leu Phe Ser Asp Gly Val Ala Phe His Asn Leu Gly
       370                 375                 380
   ttg acc att gtg gac gaa cag cac cgt ttc ggc gtt gcc caa cgc ctc     1200
   Leu Thr Ile Val Asp Glu Gln His Arg Phe Gly Val Ala Gln Arg Leu
   385                 390                 395                 400
   gcc ctc aaa aac aaa ggg cgc gaa gtc cat cag ctg atg atg tcc gcc     1248
   Ala Leu Lys Asn Lys Gly Arg Glu Val His Gln Leu Met Met Ser Ala
                   405                 410                 415
   acg ccc atc ccg cgc acg ctt gcg atg agt ttt ttc gcc gat ttg gac     1296
   Thr Pro Ile Pro Arg Thr Leu Ala Met Ser Phe Phe Ala Asp Leu Asp
               420                 425                 430
   gtg tcc gtc atc gac gaa ttg ccg ccc ggg cgc aca ccg att aaa acg     1344
   Val Ser Val Ile Asp Glu Leu Pro Pro Gly Arg Thr Pro Ile Lys Thr
           435                 440                 445
   cgc ctc gtc aac aac gtc cgc cgc gcc gaa gtc gaa ggc ttc gtc ctc     1392
   Arg Leu Val Asn Asn Val Arg Arg Ala Glu Val Glu Gly Phe Val Leu
       450                 455                 460
   ggc act tgc cga aaa ggg cgg cag gcg tat tgg gtc tgc cca ttg att    1440
   Gly Thr Cys Arg Lys Gly Arg Gln Ala Tyr Trp Val Cys Pro Leu Ile
   465                 470                 475                 480
   gaa gaa agc gaa acc ctg caa ctg caa acc gcc gcc gaa acc ctc gcc    1488
   Glu Glu Ser Glu Thr Leu Gln Leu Gln Thr Ala Ala Glu Thr Leu Ala
                   485                 490                 495
   cgg ctt cag acg gca ttg ccc gaa ctc aat atc gga ctg gta cac ggg    1536
   Arg Leu Gln Thr Ala Leu Pro Glu Leu Asn Ile Gly Leu Val His Gly
               500                 505                 510
   cgc atg aag gcc gcc gaa aag gcc gaa gtg atg gcg cgg ttt tct tcg    1584
   Arg Met Lys Ala Ala Glu Lys Ala Glu Val Met Ala Arg Phe Ser Ser
           515                 520                 525
   ggg ggg ctg aac gtc ttg gtc gcc acc acc gtc atc gaa gtc ggc gta    1632
   Gly Gly Leu Asn Val Leu Val Ala Thr Thr Val Ile Glu Val Gly Val
       530                 535                 540
   gat gtg ccc aat gcc gcc ctg atg gtc atc gaa cac gcc gag cgc atg    1680
   Asp Val Pro Asn Ala Ala Leu Met Val Ile Glu His Ala Glu Arg Met
   545                 550                 555                 560
   ggc ttg gcg cag ctt cac caa tta cgc gga cgg gta ggg cgc ggc gcg    1728
   Gly Leu Ala Gln Leu His Gln Leu Arg Gly Arg Val Gly Arg Gly Ala
                   565                 570                 575
   gcg gaa agc gtg tgc gtc ctc ctg ttt gcc gaa ccc ttg ggc gaa ctc    1776
   Ala Glu Ser Val Cys Val Leu Leu Phe Ala Glu Pro Leu Gly Glu Leu
               580                 585                 590
   gcc aaa gcg cgg ctg aaa gtc atc tac gaa cac acc gac ggc ttc gaa    1824
   Ala Lys Ala Arg Leu Lys Val Ile Tyr Glu His Thr Asp Gly Phe Glu
           595                 600                 605
   atc gcc cgc caa gac ctc aat atc cgc ggc ccg ggc gag ttt ctc ggc    1872
   Ile Ala Arg Gln Asp Leu Asn Ile Arg Gly Pro Gly Glu Phe Leu Gly
       610                 615                 620
   gcg cgc caa agc ggc gtg cct atg ctg cgt ttc gcc aaa ctc gaa gaa    1920
   Ala Arg Gln Ser Gly Val Pro Met Leu Arg Phe Ala Lys Leu Glu Glu
   625                 630                 635                 640
   gac ctg cac ctt ttg gaa cag gcg cgc gaa acc gcc ccg atg ctg att    1968
   Asp Leu His Leu Leu Glu Gln Ala Arg Glu Thr Ala Pro Met Leu Ile
                   645                 650                 655
   gag caa aac ccc gaa atc gtc gaa gcg cat ttg gca agg tgg ctt tcc    2016
   Glu Gln Asn Pro Glu Ile Val Glu Ala His Leu Ala Arg Trp Leu Ser
               660                 665                 670
   ggc agg gaa ggg tat ttg ggc gtg                                    2040
   Gly Arg Glu Gly Tyr Leu Gly Val
           675                 680
<210> 2
   <211> 680
   <212> PRT
   <213> Neisseria meningitidis
<400> 2
   Met Met Ser Pro Glu Thr Arg Lys Gln Leu Lys Ile Thr Asp Val Ser
     1               5                  10                  15
   Ala Lys Lys Leu Asp Lys Leu Asn Leu His Thr Ala Trp Asp Leu Val
               20                  25                  30
   Leu His Leu Pro Leu Arg Tyr Glu Asp Glu Thr His Ile Met Pro Ile
           35                  40                  45
   Lys Asp Ala Pro Ile Gly Val Pro Cys Gln Val Glu Gly Glu Val Ile
       50                  55                  60
   His Gln Glu Val Thr Phe Lys Pro Arg Lys Gln Leu Ile Val Gln Ile
   65                  70                  75                  80
   Ala Asp Gly Ser Gly Ser Val Leu Phe Leu Arg Phe Ile His Phe Tyr
                   85                  90                  95
   Ala Ser His Gln Lys Gln Thr Ala Ile Gly Lys Arg Ile Arg Ala Val
               100                 105                 110
   Gly Glu Ile Lys His Gly Phe Tyr Gly Asp Glu Met Ile His Pro Lys
           115                 120                 125
   Ile Arg Asp Ala Glu Gly Gly Gly Leu Ala Glu Ser Leu Thr Pro Val
       130                 135                 140
   Tyr Pro Thr Val Asn Gly Leu Asn Gln Pro Thr Leu Arg Arg Ile Ile
   145                 150                 155                 160
   Gln Thr Ala Leu Asp Val Thr Pro Leu His Asp Thr Leu Pro Asp Ala
                   165                 170                 175
   Leu Leu Cys Arg Leu Lys Leu Pro His Leu Ala Glu Ser Leu Arg Leu
               180                 185                 190
   Leu His Ser Pro Pro Pro Ser Phe Thr Ile His Gln Leu Ser Asp Gly
           195                 200                 205
   Thr Leu Pro Ala Trp Gln Arg Leu Lys Phe Asp Glu Leu Leu Ala Gln
       210                 215                 220
   Gln Leu Ser Met Arg Leu Ala Arg Gln Lys Arg Ile Gly Gly Thr Ala
   225                 230                 235                 240
   Ala Ala Leu Gly Gly Asp Gly Thr Leu Thr Gln Ala Leu Arg Gln Ala
                   245                 250                 255
   Leu Pro Phe Ala Leu Thr Asp Ala Gln Glu Lys Val Val Ser Glu Ile
               260                 265                 270
   Cys Arg Asp Met Ala Gln Thr Tyr Pro Met His Arg Leu Leu Gln Gly
           275                 280                 285
   Asp Val Gly Ser Gly Lys Thr Ile Val Ala Ala Leu Ser Ala Leu Thr
       290                 295                 300
   Ala Ile Glu Ser Gly Ala Gln Val Ala Val Met Ala Pro Thr Glu Ile
   305                 310                 315                 320
   Leu Ala Glu Gln His Phe Ile Lys Phe Lys Gln Trp Leu Glu Pro Leu
                   325                 330                 335
   Gly Ile Glu Val Val Arg Leu Phe Gly Ser Leu Arg Lys Lys Ala Lys
               340                 345                 350
   Asp Glu Ala Lys Ala Lys Leu Ala Asp Gly Ser Val Lys Ile Ala Val
           355                 360                 365
   Gly Thr His Ala Leu Phe Ser Asp Gly Val Ala Phe His Asn Leu Gly
       370                 375                 380
   Leu Thr Ile Val Asp Glu Gln His Arg Phe Gly Val Ala Gln Arg Leu
   385                 390                 395                 400
   Ala Leu Lys Asn Lys Gly Arg Glu Val His Gln Leu Met Met Ser Ala
                   405                 410                 415
   Thr Pro Ile Pro Arg Thr Leu Ala Met Ser Phe Phe Ala Asp Leu Asp
               420                 425                 430
   Val Ser Val Ile Asp Glu Leu Pro Pro Gly Arg Thr Pro Ile Lys Thr
   435 440 445
   Arg Leu Val Asn Asn Val Arg Arg Ala Glu Val Glu Gly Phe Val Leu
   450 455 460
   Gly Thr Cys Arg Lys Gly Arg Gln Ala Tyr Trp Val Cys Pro Leu Ile
   465 470 475 480
   Glu Glu Ser Glu Thr Leu Gln Leu Gln Thr Ala Ala Glu Thr Leu Ala
   485 490 495
   Arg Leu Gln Thr Ala Leu Pro Glu Leu Asn Ile Gly Leu Val His Gly
   500 505 510
   Arg Met Lys Ala Ala Glu Lys Ala Glu Val Met Ala Arg Phe Ser Ser
   515 520 525
   Gly Gly Leu Asn Val Leu Val Ala Thr Thr Val Ile Glu Val Gly Val
   530 535 540
   Asp Val Pro Asn Ala Ala Leu Met Val Ile Glu His Ala Glu Arg Met
   545 550 555 560
   Gly Leu Ala Gln Leu His Gln Leu Arg Gly Arg Val Gly Arg Gly Ala
   565 570 575
   Ala Glu Ser Val Cys Val Leu Leu Phe Ala Glu Pro Leu Gly Glu Leu
   580 585 590
   Ala Lys Ala Arg Leu Lys Val Ile Tyr Glu His Thr Asp Gly Phe Glu
   595 600 605
   Ile Ala Arg Gln Asp Leu Asn Ile Arg Gly Pro Gly Glu Phe Leu Gly
   610 615 620
   Ala Arg Gln Ser Gly Val Pro Met Leu Arg Phe Ala Lys Leu Glu Glu
   625 630 635 640
   Asp Leu His Leu Leu Glu Gln Ala Arg Glu Thr Ala Pro Met Leu Ile
   645 650 655
   Glu Gln Asn Pro Glu Ile Val Glu Ala His Leu Ala Arg Trp Leu Ser
   660 665 670
   Gly Arg Glu Gly Tyr Leu Gly Val
   675 680
<210> 3
   <211> 1257
   <212> DNA
   <213> Neisseria meningitidis
<220>
   <221> CDS
   <222> (1)..(1257)
<400> 3
   atg cac gtc tcc gaa tta caa acc ctg cac att tcc aaa ctc tta gaa    48
   Met His Val Ser Glu Leu Gln Thr Leu His Ile Ser Lys Leu Leu Glu
   1               5                   10                  15
   ttg gcg gaa gaa cac ggc atc gaa aac gcc aac cga ttc cgc aaa caa    96
   Leu Ala Glu Glu His Gly Ile Glu Asn Ala Asn Arg Phe Arg Lys Gln
               20                  25                  30
   gat ctc gta ttt gcc atc gtc cgc cag atg atg aaa aaa ggc gag ggt    144
   Asp Leu Val Phe Ala Ile Val Arg Gln Met Met Lys Lys Gly Glu Gly
           35                  40                  45
   ttc acc tgc tcc ggc acg ctt gaa atc ctg ccc gac ggc ttc ggc ttc    192
   Phe Thr Cys Ser Gly Thr Leu Glu Ile Leu Pro Asp Gly Phe Gly Phe
       50                  55                  60
   ctc cgc agc gcg gac acg tcc tat ctt gcc ggc ccc gac gac atc tat    240
   Leu Arg Ser Ala Asp Thr Ser Tyr Leu Ala Gly Pro Asp Asp Ile Tyr
   65                  70                  75                  80
   gtc tcg ccc acc caa atc cgc cgc ttc aac ctg cat acg ggc gac acc    288
   Val Ser Pro Thr Gln Ile Arg Arg Phe Asn Leu His Thr Gly Asp Thr
                   85                  90                  95
   atc gaa gga agc gtg cgc gtc cca aaa gac aac gaa cgc tat ttt gcc    336
   Ile Glu Gly Ser Val Arg Val Pro Lys Asp Asn Glu Arg Tyr Phe Ala
               100                 105                 110
   ctg gtc agg ctt gat acc atc aac ggc gac cac ccg gaa gta tgc cgc    384
   Leu Val Arg Leu Asp Thr Ile Asn Gly Asp His Pro Glu Val Cys Arg
           115                 120                 125
   cat aaa atc ctg ttt gaa aac ctg acc ccg ctg ttt ccg acc gaa cag    432
   His Lys Ile Leu Phe Glu Asn Leu Thr Pro Leu Phe Pro Thr Glu Gln
       130                 135                 140
   ttg aag ctg gaa cgc gac tta aag tcc gaa gaa aac ctg acc gga cgt    480
   Leu Lys Leu Glu Arg Asp Leu Lys Ser Glu Glu Asn Leu Thr Gly Arg
   145                 150                 155                 160
   gcc atc gac ctg att tcc cct atc ggc aaa ggt cag cgc gcc ctc ttg    528
   Ala Ile Asp Leu Ile Ser Pro Ile Gly Lys Gly Gln Arg Ala Leu Leu
                   165                 170                 175
   gtt gcc ccg ccc aaa agc ggt aaa acc gtg atg ctg caa aac att gcc    576
   Val Ala Pro Pro Lys Ser Gly Lys Thr Val Met Leu Gln Asn Ile Ala
               180                 185                 190
   cac gcc gtt acc tca aac tat ccc gaa gtc gaa ctc atc gtc ctc ttg    624
   His Ala Val Thr Ser Asn Tyr Pro Glu Val Glu Leu Ile Val Leu Leu
           195                 200                 205
   att gac gag cgt ccc gaa gaa gta acc gaa atg agc cgt tcc gtc cgc    672
   Ile Asp Glu Arg Pro Glu Glu Val Thr Glu Met Ser Arg Ser Val Arg
       210                 215                 220
   ggc gaa gtg gtt tcc tcc acc ttt gac gaa ccg gcg caa cgc cac gtc    720
   Gly Glu Val Val Ser Ser Thr Phe Asp Glu Pro Ala Gln Arg His Val
   225                 230                 235                 240
   caa gtt gcc gaa atg gtg ctt gaa aaa gcc aag cgt atg gta gaa cac    768
   Gln Val Ala Glu Met Val Leu Glu Lys Ala Lys Arg Met Val Glu His
                   245                 250                 255
   aaa aaa gac gtg gtc atc ctg ctg gat tcg att acc cgc ctt gcc cgc    816
   Lys Lys Asp Val Val Ile Leu Leu Asp Ser Ile Thr Arg Leu Ala Arg
               260                 265                 270
   gcc tac aat acc gtc gtg cct acc tcg ggc aaa atc cta acc ggc ggt    864
   Ala Tyr Asn Thr Val Val Pro Thr Ser Gly Lys Ile Leu Thr Gly Gly
           275                 280                 285
   gtc gat gcc aac gca ctg cac cgc ccc aaa cgt ttc ttc ggc gcg gcg    912
   Val Asp Ala Asn Ala Leu His Arg Pro Lys Arg Phe Phe Gly Ala Ala
       290                 295                 300
   cgc aac gtg gaa gaa ggc ggt tcg ctg acc atc atc gcc acc gca ttg    960
   Arg Asn Val Glu Glu Gly Gly Ser Leu Thr Ile Ile Ala Thr Ala Leu
   305                 310                 315                 320
   gtt gaa acc ggc agc cgt atg gac gat gtg att tac gaa gaa ttc aaa   1008
   Val Glu Thr Gly Ser Arg Met Asp Asp Val Ile Tyr Glu Glu Phe Lys
                   325                 330                 335
   ggc acc ggc aat atg gaa ttg cac ctt gac cgc cgt atg gcg gaa aaa     1056
   Gly Thr Gly Asn Met Glu Leu His Leu Asp Arg Arg Met Ala Glu Lys
                   340                 345                 350
   cgc ctc ttc ccc gcc atc aac atc aac aaa tcc ggc acg cgc cgc gaa     1104
   Arg Leu Phe Pro Ala Ile Asn Ile Asn Lys Ser Gly Thr Arg Arg Glu
               355                 360                 365
   gag ctg ctt gtg ccg aac gac cag ttg caa cgt atg tgg ctc tta cgc     1152
   Glu Leu Leu Val Pro Asn Asp Gln Leu Gln Arg Met Trp Leu Leu Arg
           370                 375                 380
   aag ttc ctg cac ccg atg gac gaa atc gag gca gcc gaa ttt tta atc     1200
   Lys Phe Leu His Pro Met Asp Glu Ile Glu Ala Ala Glu Phe Leu Ile
   385                 390                 395                 400
   ggg aaa atc aaa gcc tct aaa aac aat gat gat ttc ttt gaa ctg atg     1248
   Gly Lys Ile Lys Ala Ser Lys Asn Asn Asp Asp Phe Phe Glu Leu Met
                       405                 410                 415
   cgc ggc aaa                                                         1257
   Arg Gly Lys
<210> 4
   <211> 419
   <212> PRT
   <213> Neisseria meningitidis
<400> 4
   Met His Val Ser Glu Leu Gln Thr Leu His Ile Ser Lys Leu Leu Glu
    1               5                  10                  15
   Leu Ala Glu Glu His Gly Ile Glu Asn Ala Asn Arg Phe Arg Lys Gln
               20                  25                  30
   Asp Leu Val Phe Ala Ile Val Arg Gln Met Met Lys Lys Gly Glu Gly
           35                  40                  45
   Phe Thr Cys Ser Gly Thr Leu Glu Ile Leu Pro Asp Gly Phe Gly Phe
       50                  55                  60
   Leu Arg Ser Ala Asp Thr Ser Tyr Leu Ala Gly Pro Asp Asp Ile Tyr
   65                  70                  75                  80
   Val Ser Pro Thr Gln Ile Arg Arg Phe Asn Leu His Thr Gly Asp Thr
                   85                  90                  95
   Ile Glu Gly Ser Val Arg Val Pro Lys Asp Asn Glu Arg Tyr Phe Ala
               100                 105                 110
   Leu Val Arg Leu Asp Thr Ile Asn Gly Asp His Pro Glu Val Cys Arg
           115                 120                 125
   His Lys Ile Leu Phe Glu Asn Leu Thr Pro Leu Phe Pro Thr Glu Gln
       130                 135                 140
   Leu Lys Leu Glu Arg Asp Leu Lys Ser Glu Glu Asn Leu Thr Gly Arg
   145                 150                 155                 160
   Ala Ile Asp Leu Ile Ser Pro Ile Gly Lys Gly Gln Arg Ala Leu Leu
                   165                 170                 175
   Val Ala Pro Pro Lys Ser Gly Lys Thr Val Met Leu Gln Asn Ile Ala
               180                 185                 190
   His Ala Val Thr Ser Asn Tyr Pro Glu Val Glu Leu Ile Val Leu Leu
           195                 200                 205
   Ile Asp Glu Arg Pro Glu Glu Val Thr Glu Met Ser Arg Ser Val Arg
       210                 215                 220
   Gly Glu Val Val Ser Ser Thr Phe Asp Glu Pro Ala Gln Arg His Val
   225                 230                 235                 240
   Gln Val Ala Glu Met Val Leu Glu Lys Ala Lys Arg Met Val Glu His
                   245                 250                 255
   Lys Lys Asp Val Val Ile Leu Leu Asp Ser Ile Thr Arg Leu Ala Arg
               260                 265                 270
   Ala Tyr Asn Thr Val Val Pro Thr Ser Gly Lys Ile Leu Thr Gly Gly
           275                 280                 285
   Val Asp Ala Asn Ala Leu His Arg Pro Lys Arg Phe Phe Gly Ala Ala
       290                 295                 300
   Arg Asn Val Glu Glu Gly Gly Ser Leu Thr Ile Ile Ala Thr Ala Leu
   305                 310                 315                 320
   Val Glu Thr Gly Ser Arg Met Asp Asp Val Ile Tyr Glu Glu Phe Lys
                   325                 330                 335
   Gly Thr Gly Asn Met Glu Leu His Leu Asp Arg Arg Met Ala Glu Lys
               340                 345                 350
   Arg Leu Phe Pro Ala Ile Asn Ile Asn Lys Ser Gly Thr Arg Arg Glu
           355                 360                 365
   Glu Leu Leu Val Pro Asn Asp Gln Leu Gln Arg Met Trp Leu Leu Arg
       370                 375                 380
   Lys Phe Leu His Pro Met Asp Glu Ile Glu Ala Ala Glu Phe Leu Ile
   385                 390                 395                 400
   Gly Lys Ile Lys Ala Ser Lys Asn Asn Asp Asp Phe Phe Glu Leu Met
                   405                 410                 415
   Arg Gly Lys
<210> 5
   <211> 599
   <212> DNA
   <213> Neisseria meningitidis
<220>
   <221> CDS
   <222> (1)..(597)
<400> 5
   atg aac aat tta atc aaa atc aac ctc ctc ccc tac agg gaa gag atg    48
   Met Asn Asn Leu Ile Lys Ile Asn Leu Leu Pro Tyr Arg Glu Glu Met
     1               5                  10                  15
   aac aag cgc aaa cag cag cag ttt aaa acg ctg atg tac ggt gcc gtg    96
   Asn Lys Arg Lys Gln Gln Gln Phe Lys Thr Leu Met Tyr Gly Ala Val
                20                  25                  30
   ctg acg ggc gtt gcc gcc gtt gcg gca acc tac ctg ttt atc gac aat   144
   Leu Thr Gly Val Ala Ala Val Ala Ala Thr Tyr Leu Phe Ile Asp Asn
            35                  40                  45
   atg atc aat aac cag tcg gaa aga aac acg ctg ctg gaa acc tcc atc   192
   Met Ile Asn Asn Gln Ser Glu Arg Asn Thr Leu Leu Glu Thr Ser Ile
        50                  55                  60
   gca cac ttg gat acc gag ctg tcg gaa ata caa aag ctc aaa cag gaa   240
   Ala His Leu Asp Thr Glu Leu Ser Glu Ile Gln Lys Leu Lys Gln Glu
    65                  70                  75                  80
   aaa gat gcc ttc ctg att aag aaa aac aaa atc gag gag ctc cag ctc   288
   Lys Asp Ala Phe Leu Ile Lys Lys Asn Lys Ile Glu Glu Leu Gln Leu
                   85                  90                  95
   aaa cgc ctc caa gcc gca aaa atc ctc gac agc ctg aat gag gcc gtc      336
   Lys Arg Leu Gln Ala Ala Lys Ile Leu Asp Ser Leu Asn Glu Ala Val
               100                 105                 110
   ccc gga agc acc tac ctg acc tcg ctg gat gcc gtt acc gcc gac tct      384
   Pro Gly Ser Thr Tyr Leu Thr Ser Leu Asp Ala Val Thr Ala Asp Ser
           115                 120                 125
   tat cgg ctc agc ggc agg aca tcc agc gac aac cgc gtt gcc gcc atg      432
   Tyr Arg Leu Ser Gly Arg Thr Ser Ser Asp Asn Arg Val Ala Ala Met
       130                 135                 140
   atg agg gcg atg ccc aat acc ggc ata ttc aag caa ccc gaa ttg tta      480
   Met Arg Ala Met Pro Asn Thr Gly Ile Phe Lys Gln Pro Glu Leu Leu
   145                 150                 155                 160
   agc atc aag aaa aac aat tcg cat caa gaa ttt acc ctt cag gca aca      528
   Ser Ile Lys Lys Asn Asn Ser His Gln Glu Phe Thr Leu Gln Ala Thr
                   165                 170                 175
   tta caa ccc atc gta aag gcg gcc gaa tcc aaa gag aat ccg gct tcg      576
   Leu Gln Pro Ile Val Lys Ala Ala Glu Ser Lys Glu Asn Pro Ala Ser
               180                 185                 190
   gga aac gca cag gag gca aac tg                                       599
   Gly Asn Ala Gln Glu Ala Asn
           195
<210> 6
   <211> 199
   <212> PRT
   <213> Neisseria meningitidis
<400> 6
   Met Asn Asn Leu Ile Lys Ile Asn Leu Leu Pro Tyr Arg Glu Glu Met
   1 5 10 15
   Asn Lys Arg Lys Gln Gln Gln Phe Lys Thr Leu Met Tyr Gly Ala Val
   20 25 30
   Leu Thr Gly Val Ala Ala Val Ala Ala Thr Tyr Leu Phe Ile Asp Asn
   35 40 45
   Met Ile Asn Asn Gln Ser Glu Arg Asn Thr Leu Leu Glu Thr Ser Ile
   50 55 60
   Ala His Leu Asp Thr Glu Leu Ser Glu Ile Gln Lys Leu Lys Gln Glu
   65 70 75 80
   Lys Asp Ala Phe Leu Ile Lys Lys Asn Lys Ile Glu Glu Leu Gln Leu
   85 90 95
   Lys Arg Leu Gln Ala Ala Lys Ile Leu Asp Ser Leu Asn Glu Ala Val
   100 105 110
   Pro Gly Ser Thr Tyr Leu Thr Ser Leu Asp Ala Val Thr Ala Asp Ser
   115 120 125
   Tyr Arg Leu Ser Gly Arg Thr Ser Ser Asp Asn Arg Val Ala Ala Met
   130 135 140
   Met Arg Ala Met Pro Asn Thr Gly Ile Phe Lys Gln Pro Glu Leu Leu
   145 150 155 160
   Ser Ile Lys Lys Asn Asn Ser His Gln Glu Phe Thr Leu Gln Ala Thr
   165 170 175
   Leu Gln Pro Ile Val Lys Ala Ala Glu Ser Lys Glu Asn Pro Ala Ser
   180 185 190
   Gly Asn Ala Gln Glu Ala Asn
   195
<210> 7
   <211> 1773
   <212> DNA
   <213> Neisseria meningitidis
<220>
   <221> CDS
   <222> (1)..(1773)
<400> 7
   atg agt atc gtg ctg cac ggc gtg gcg gcg ggc aaa ggc att gcc gtc    48
   Met Ser Ile Val Leu His Gly Val Ala Ala Gly Lys Gly Ile Ala Val
    1               5                  10                  15
   ggt tgc gcc cac ctg att gcg cgc ggt acg gag gaa gtg ccg cag tat    96
   Gly Cys Ala His Leu Ile Ala Arg Gly Thr Glu Glu Val Pro Gln Tyr
               20                  25                  30
   gat gtt gcg gag gcg gac acc gat gcc gaa gcc gaa cgt ttc gat gcc     144
   Asp Val Ala Glu Ala Asp Thr Asp Ala Glu Ala Glu Arg Phe Asp Ala
               35                  40                  45
   gcc gtc aaa gcc acg cgc aaa gag ttg gaa cag ctc cgc agc gcg att     192
   Ala Val Lys Ala Thr Arg Lys Glu Leu Glu Gln Leu Arg Ser Ala Ile
           50                  55                  60
   ccc gaa aac gcc ccg acc gag ttg ggc gcg ttc atc tcg ctg cac ctg     240
   Pro Glu Asn Ala Pro Thr Glu Leu Gly Ala Phe Ile Ser Leu His Leu
    65                  70                  75                  80
   atg ctc ttg acc gat gtt acc ttg tcg cgc gaa ccc gtc gat att tta     288
   Met Leu Leu Thr Asp Val Thr Leu Ser Arg Glu Pro Val Asp Ile Leu
                        85                  90                  95
   agg gaa caa aaa atc aac gcc gag tgg gca ttg aag cag cag agc gac     336
   Arg Glu Gln Lys Ile Asn Ala Glu Trp Ala Leu Lys Gln Gln Ser Asp
                  100                 105                 110
   aaa ctc gcc gcc caa ttc gac aat atg gac gat gcc tat ttg cgc gaa     384
   Lys Leu Ala Ala Gln Phe Asp Asn Met Asp Asp Ala Tyr Leu Arg Glu
           115                 120                 125
   cgc aag cag gat atg ctg caa gtc gtc cgc cgc atc cac aac aac ctg     432
   Arg Lys Gln Asp Met Leu Gln Val Val Arg Arg Ile His Asn Asn Leu
       130                 135                 140
   atc ggg cag ggc aac gag ttg gaa gtt gcc gac aac ctg ttt gac gaa     480
   Ile Gly Gln Gly Asn Glu Leu Glu Val Ala Asp Asn Leu Phe Asp Glu
   145                 150                 155                 160
   acc gtt ctg att gca aac gac ctt tcg ccc gcc gac acg gtt ttg ttt     528
   Thr Val Leu Ile Ala Asn Asp Leu Ser Pro Ala Asp Thr Val Leu Phe
                   165                 170                 175
   aaa gag cag cgc att gcc gcc ttc gtt acc gat gcc ggc ggc ccc acc     576
   Lys Glu Gln Arg Ile Ala Ala Phe Val Thr Asp Ala Gly Gly Pro Thr
               180                 185                 190
   ggg cat acg gcg att ttg ggc agg agc ttg gac atc ccg tcc gtc gtc     624
   Gly His Thr Ala Ile Leu Gly Arg Ser Leu Asp Ile Pro Ser Val Val
           195                 200                 205
   ggg ctg cac aac gcg cgc aaa ctg att acc gag ggc gaa acg gtc att     672
   Gly Leu His Asn Ala Arg Lys Leu Ile Thr Glu Gly Glu Thr Val Ile
       210                 215                 220
   gtg gac ggt atc aac ggc gtg ttg att atc gcg ccg gat gag tcg gtg      720
   Val Asp Gly Ile Asn Gly Val Leu Ile Ile Ala Pro Asp Glu Ser Val
   225                 230                 235                 240
   ttg aac gaa tac cgc cgc cgt gcc cgc gaa tac cgc agc cac aaa cgc      768
   Leu Asn Glu Tyr Arg Arg Arg Ala Arg Glu Tyr Arg Ser His Lys Arg
                   245                 250                 255
   gat ttg aac aag ctc aaa aaa acc gcc gcc gcc acc gct gac ggg gtc      816
   Asp Leu Asn Lys Leu Lys Lys Thr Ala Ala Ala Thr Ala Asp Gly Val
               260                 265                 270
   tgc atc gag ctt gtg ggc aat ata gaa tcc gcc gaa gac gtg aaa ccg      864
   Cys Ile Glu Leu Val Gly Asn Ile Glu Ser Ala Glu Asp Val Lys Pro
           275                 280                 285
   ctg cac aac ctc ggc gca gac ggc atc ggg ctg ttc cgc agc gag ttt      912
   Leu His Asn Leu Gly Ala Asp Gly Ile Gly Leu Phe Arg Ser Glu Phe
       290                 295                 300
   ctt tac ctg aac cgc gat acg atg ccg tct gaa gac gag cag tac gaa      960
   Leu Tyr Leu Asn Arg Asp Thr Met Pro Ser Glu Asp Glu Gln Tyr Glu
   305                 310                 315                 320
   gtg tac agc gcg att gtc aaa aaa atg aaa ggc aaa agc gta acg ata      1008
   Val Tyr Ser Ala Ile Val Lys Lys Met Lys Gly Lys Ser Val Thr Ile
                   325                 330                 335
   cgg aca gtc gat ttg ggt gtg gac aaa aac ccg cgc tgg ttc ggg aaa      1056
   Arg Thr Val Asp Leu Gly Val Asp Lys Asn Pro Arg Trp Phe Gly Lys
               340                 345                 350
   aac agc acg ccc aac ggc agc ctc aac ccc gcg ctg ggc atg acc ggc      1104
   Asn Ser Thr Pro Asn Gly Ser Leu Asn Pro Ala Leu Gly Met Thr Gly
           355                 360                 365
   atc cgc ctg tgc ctt gcc gaa ccg gtc atg ttc cgc acc cag atg cgc      1152
   Ile Arg Leu Cys Leu Ala Glu Pro Val Met Phe Arg Thr Gln Met Arg
       370                 375                 380
   gcc atc ctc cgc gcc gcc gcc cac ggc ccc gtg cgg atg atg tgg ccg      1200
   Ala Ile Leu Arg Ala Ala Ala His Gly Pro Val Arg Met Met Trp Pro
   385                 390                 395                 400
   atg att acc tcc gta tcc gaa gtg cgc cag tgc ctc atc cac ctc gac      1248
   Met Ile Thr Ser Val Ser Glu Val Arg Gln Cys Leu Ile His Leu Asp
                   405                 410                 415
   acc gcg caa cgc cag ctt gcc gaa cgc ggc gat gcc ttc ggt aaa gtc    1296
   Thr Ala Gln Arg Gln Leu Ala Glu Arg Gly Asp Ala Phe Gly Lys Val
               420                 425                 430
   ggc atc ggc tgt atg att gaa att ccg tct gcc gcg ctg acc gtc ggc    1344
   Gly Ile Gly Cys Met Ile Glu Ile Pro Ser Ala Ala Leu Thr Val Gly
           435                 440                 445
   agt att ttg aaa ctg gtc gat ttc atc tcc gtc ggt acc aac gac ctg    1392
   Ser Ile Leu Lys Leu Val Asp Phe Ile Ser Val Gly Thr Asn Asp Leu
       450                 455                 460
   att caa tac atc ttg tcc gtc gat cgc ggc gac gac agc gtc agc cac    1440
   Ile Gln Tyr Ile Leu Ser Val Asp Arg Gly Asp Asp Ser Val Ser His
   465                 470                 475                 480
   ctc tac cag ccc ggc cat ccc gcc gtg ctg aaa atg ctg caa cac gtc    1488
   Leu Tyr Gln Pro Gly His Pro Ala Val Leu Lys Met Leu Gln His Val
                   485                 490                 495
   atc cgt acc gcc aac cgc atg gac aaa gac gta tcc gta tgc ggc gag    1536
   Ile Arg Thr Ala Asn Arg Met Asp Lys Asp Val Ser Val Cys Gly Glu
               500                 505                 510
   atg gcg ggc gat acc gcg ttt acc cgc gtt tta ttg ggt atg ggg ctg    1584
   Met Ala Gly Asp Thr Ala Phe Thr Arg Val Leu Leu Gly Met Gly Leu
           515                 520                 525
   cgc cgt ttt tcc atg aac ccc aac aac atc ctg ccc gtc aaa aac atc    1632
   Arg Arg Phe Ser Met Asn Pro Asn Asn Ile Leu Pro Val Lys Asn Ile
       530                 535                 540
   att ctg cac agc aat gtc gga cag ctc gaa agc gat att gtg aaa gtc    1680
   Ile Leu His Ser Asn Val Gly Gln Leu Glu Ser Asp Ile Val Lys Val
   545                 550                 555                 560
   atc cgc tgc gaa gac gaa gag aag tcg gaa aag ctg atc aaa cag atg    1728
   Ile Arg Cys Glu Asp Glu Glu Lys Ser Glu Lys Leu Ile Lys Gln Met
                   565                 570                 575
   aac agc gtg tct gtc gag gaa gaa gcc gac ttc aag ggg cgg aaa        1773
   Asn Ser Val Ser Val Glu Glu Glu Ala Asp Phe Lys Gly Arg Lys
               580                 585                 590
<210> 8
   <211> 591
<212> PRT
   <213> Neisseria meningitidis
<400> 8
   Met Ser Ile Val Leu His Gly Val Ala Ala Gly Lys Gly Ile Ala Val
   1 5 10 15
   Gly Cys Ala His Leu Ile Ala Arg Gly Thr Glu Glu Val Pro Gln Tyr
   20 25 30
   Asp Val Ala Glu Ala Asp Thr Asp Ala Glu Ala Glu Arg Phe Asp Ala
   35 40 45
   Ala Val Lys Ala Thr Arg Lys Glu Leu Glu Gln Leu Arg Ser Ala Ile
   50 55 60
   Pro Glu Asn Ala Pro Thr Glu Leu Gly Ala Phe Ile Ser Leu His Leu
   65 70 75 80
   Met Leu Leu Thr Asp Val Thr Leu Ser Arg Glu Pro Val Asp Ile Leu
   85 90 95
   Arg Glu Gln Lys Ile Asn Ala Glu Trp Ala Leu Lys Gln Gln Ser Asp
   100 105 110
   Lys Leu Ala Ala Gln Phe Asp Asn Met Asp Asp Ala Tyr Leu Arg Glu
   115 120 125
   Arg Lys Gln Asp Met Leu Gln Val Val Arg Arg Ile His Asn Asn Leu
   130 135 140
   Ile Gly Gln Gly Asn Glu Leu Glu Val Ala Asp Asn Leu Phe Asp Glu
   145 150 155 160
   Thr Val Leu Ile Ala Asn Asp Leu Ser Pro Ala Asp Thr Val Leu Phe
   165 170 175
   Lys Glu Gln Arg Ile Ala Ala Phe Val Thr Asp Ala Gly Gly Pro Thr
   180 185 190
   Gly His Thr Ala Ile Leu Gly Arg Ser Leu Asp Ile Pro Ser Val Val
   195 200 205
   Gly Leu His Asn Ala Arg Lys Leu Ile Thr Glu Gly Glu Thr Val Ile
   210 215 220
   Val Asp Gly Ile Asn Gly Val Leu Ile Ile Ala Pro Asp Glu Ser Val
   225 230 235 240
   Leu Asn Glu Tyr Arg Arg Arg Ala Arg Glu Tyr Arg Ser His Lys Arg
   245 250 255
   Asp Leu Asn Lys Leu Lys Lys Thr Ala Ala Ala Thr Ala Asp Gly Val
   260 265 270
   Cys Ile Glu Leu Val Gly Asn Ile Glu Ser Ala Glu Asp Val Lys Pro
   275 280 285
   Leu His Asn Leu Gly Ala Asp Gly Ile Gly Leu Phe Arg Ser Glu Phe
   290 295 300
   Leu Tyr Leu Asn Arg Asp Thr Met Pro Ser Glu Asp Glu Gln Tyr Glu
   305 310 315 320
   Val Tyr Ser Ala Ile Val Lys Lys Met Lys Gly Lys Ser Val Thr Ile
   325 330 335
   Arg Thr Val Asp Leu Gly Val Asp Lys Asn Pro Arg Trp Phe Gly Lys
   340 345 350
   Asn Ser Thr Pro Asn Gly Ser Leu Asn Pro Ala Leu Gly Met Thr Gly
   355 360 365
   Ile Arg Leu Cys Leu Ala Glu Pro Val Met Phe Arg Thr Gln Met Arg
   370 375 380
   Ala Ile Leu Arg Ala Ala Ala His Gly Pro Val Arg Met Met Trp Pro
   385 390 395 400
   Met Ile Thr Ser Val Ser Glu Val Arg Gln Cys Leu Ile His Leu Asp
   405 410 415
   Thr Ala Gln Arg Gln Leu Ala Glu Arg Gly Asp Ala Phe Gly Lys Val
   420 425 430
   Gly Ile Gly Cys Met Ile Glu Ile Pro Ser Ala Ala Leu Thr Val Gly
   435 440 445
   Ser Ile Leu Lys Leu Val Asp Phe Ile Ser Val Gly Thr Asn Asp Leu
   450 455 460
   Ile Gln Tyr Ile Leu Ser Val Asp Arg Gly Asp Asp Ser Val Ser His
   465 470 475 480
   Leu Tyr Gln Pro Gly His Pro Ala Val Leu Lys Met Leu Gln His Val
   485 490 495
   Ile Arg Thr Ala Asn Arg Met Asp Lys Asp Val Ser Val Cys Gly Glu
               500                 505                 510
   Met Ala Gly Asp Thr Ala Phe Thr Arg Val Leu Leu Gly Met Gly Leu
           515                 520                 525
   Arg Arg Phe Ser Met Asn Pro Asn Asn Ile Leu Pro Val Lys Asn Ile
       530                 535                 540
   Ile Leu His Ser Asn Val Gly Gln Leu Glu Ser Asp Ile Val Lys Val
   545                 550                 555                 560
   Ile Arg Cys Glu Asp Glu Glu Lys Ser Glu Lys Leu Ile Lys Gln Met
                   565                 570                 575
   Asn Ser Val Ser Val Glu Glu Glu Ala Asp Phe Lys Gly Arg Lys
               580                 585                 590
<210> 9
   <211> 1572
   <212> DNA
   <213> Neisseria meningitidis
<220>
   <221> CDS
   <222> (1)..(1572)
<400> 9
   atg ttc aga cgg tat ctt ccg aac aga cag atg aat atg gtt tcc aaa     48
   Met Phe Arg Arg Tyr Leu Pro Asn Arg Gln Met Asn Met Val Ser Lys
    1               5                  10                  15
   ctg gac aaa tac tgg cag cac ccc gcc ctc tac tgg cct ttg ctc atc     96
   Leu Asp Lys Tyr Trp Gln His Pro Ala Leu Tyr Trp Pro Leu Leu Ile
               20                  25                  30
   ctt ttt gcc gcc gcc acc ccc ttt acc ttc gca ccc tac tac cac ttt    144
   Leu Phe Ala Ala Ala Thr Pro Phe Thr Phe Ala Pro Tyr Tyr His Phe
           35                  40                  45
   tgg ctg atg ccc ttg att ttc ggt gcc ttc gtc cgc ctc atc gaa ctg    192
   Trp Leu Met Pro Leu Ile Phe Gly Ala Phe Val Arg Leu Ile Glu Leu
       50                  55                  60
   cgt ccg cgt ttt gct gtc tct tcc gcc tac ctg ttc ggc ctg acc gca    240
   Arg Pro Arg Phe Ala Val Ser Ser Ala Tyr Leu Phe Gly Leu Thr Ala
   65 70 75 80
   tac acg aca cag ttc tac tgg ata cac acc gcc ctg cac gac gtt tcc 288
   Tyr Thr Thr Gln Phe Tyr Trp Ile His Thr Ala Leu His Asp Val Ser
   85 90 95
   ggc ctg ccc gac ctc tat gcc gta ccg ctg acc ttc cta ctc ccc gcc 336
   Gly Leu Pro Asp Leu Tyr Ala Val Pro Leu Thr Phe Leu Leu Pro Ala
   100 105 110
   tac ctt gcc ctt tat ccg gca ctg tgt ttc tgg ctg tgg aaa aaa ttt 384
   Tyr Leu Ala Leu Tyr Pro Ala Leu Cys Phe Trp Leu Trp Lys Lys Phe
   115 120 125
   acc ctg cct cgg ggc ata aaa atc ggt ttg gta ctg ccc atc ctg tgg 432
   Thr Leu Pro Arg Gly Ile Lys Ile Gly Leu Val Leu Pro Ile Leu Trp
   130 135 140
   aca ctg acc gag ttt gcc cgc gaa cgt ttc ctg acc gga ttc ggc tgg 480
   Thr Leu Thr Glu Phe Ala Arg Glu Arg Phe Leu Thr Gly Phe Gly Trp
   145 150 155 160
   ggc gca atc ggc tac tcc caa atc acc ccg gac agc ccg ctc gcc ggc 528
   Gly Ala Ile Gly Tyr Ser Gln Ile Thr Pro Asp Ser Pro Leu Ala Gly
   165 170 175
   ttt gcc cca ttt ggc ggc atc cac atg gtt aca ctg gca acc gcc ttt 576
   Phe Ala Pro Phe Gly Gly Ile His Met Val Thr Leu Ala Thr Ala Phe
   180 185 190
   ctc ggt gtc tgg ctg gtt ttg gcg agt gat aac acc gca cgt tcg ggc 624
   Leu Gly Val Trp Leu Val Leu Ala Ser Asp Asn Thr Ala Arg Ser Gly
   195 200 205
   aaa cgc ctg ctt cca att atc ctg att gcc gcc ctg ctt gcc gca ggc 672
   Lys Arg Leu Leu Pro Ile Ile Leu Ile Ala Ala Leu Leu Ala Ala Gly
   210 215 220
   tac acc gcc cga caa acc gac ttc acc cgc ccc gac ggc agc cgc agc 720
   Tyr Thr Ala Arg Gln Thr Asp Phe Thr Arg Pro Asp Gly Ser Arg Ser
   225 230 235 240
   acc gtc gcc ctg ctt caa ggc aac atc gac caa acc ctc aaa tgg cgt 768
   Thr Val Ala Leu Leu Gln Gly Asn Ile Asp Gln Thr Leu Lys Trp Arg
   245 250 255
   gaa gac caa gtc atc ccg acc ata cag aaa tat tac gaa cag gtc ggc 816
   Glu Asp Gln Val Ile Pro Thr Ile Gln Lys Tyr Tyr Glu Gln Val Gly
               260                 265                 270
   aaa acc act gcc gac atc gtc atc ctg ccc gaa acc gcc atc ccc gtc      864
   Lys Thr Thr Ala Asp Ile Val Ile Leu Pro Glu Thr Ala Ile Pro Val
           275                 280                 285
   atg cgc caa aac ctg ccg gaa aac ata ctg gca aaa ttt gcc gaa cag      912
   Met Arg Gln Asn Leu Pro Glu Asn Ile Leu Ala Lys Phe Ala Glu Gln
       290                 295                 300
   gcg caa aac aac ggc agc gcg ctc gcc gtc ggc atc agc caa tac act      960
   Ala Gln Asn Asn Gly Ser Ala Leu Ala Val Gly Ile Ser Gln Tyr Thr
   305                 310                 315                 320
   tcg gac ggc aac ggt tac gaa aac gcc gtc atc aac ctg acc ggt tat     1008
   Ser Asp Gly Asn Gly Tyr Glu Asn Ala Val Ile Asn Leu Thr Gly Tyr
                   325                 330                 335
   cag gaa aac aat cag gac ggc atc ccc tac tac gcc aaa aac cac ctc     1056
   Gln Glu Asn Asn Gln Asp Gly Ile Pro Tyr Tyr Ala Lys Asn His Leu
               340                 345                 350
   gtc ccc ttc ggc gaa tac aaa ccg ctg ccc ttc ctg acc acg ccg ctt     1104
   Val Pro Phe Gly Glu Tyr Lys Pro Leu Pro Phe Leu Thr Thr Pro Leu
           355                 360                 365
   tac aaa atg atg aat atg ccc ctt tcc gac ttc cgc aaa ggc ggc ggt     1152
   Tyr Lys Met Met Asn Met Pro Leu Ser Asp Phe Arg Lys Gly Gly Gly
       370                 375                 380
   aag caa tcc gcc ctg ctg atg aaa aac caa aaa atc gcc ttc aac atc     1200
   Lys Gln Ser Ala Leu Leu Met Lys Asn Gln Lys Ile Ala Phe Asn Ile
   385                 390                 395                 400
   tgt tac gaa gac gga ttc ggc gac gaa ctg att gcc gcc gcc aaa gat     1248
   Cys Tyr Glu Asp Gly Phe Gly Asp Glu Leu Ile Ala Ala Ala Lys Asp
                   405                 410                 415
   gcc aca ctg ctt gcc aat gcc agc aat atg gcg tgg tac gga aaa tcc     1296
   Ala Thr Leu Leu Ala Asn Ala Ser Asn Met Ala Trp Tyr Gly Lys Ser
               420                 425                 430
   aac gcc atg tac cag cac ctc caa caa tcg cag gcg cgg gct atg gaa     1344
   Asn Ala Met Tyr Gln His Leu Gln Gln Ser Gln Ala Arg Ala Met Glu
           435                 440                 445
   ctc gga cgc tat atg gtc cgt gcc acc aac acc ggc gca acc gcc atc     1392
   Leu Gly Arg Tyr Met Val Arg Ala Thr Asn Thr Gly Ala Thr Ala Ile
       450                 455                 460
   atc tcc ccc aaa ggc aac atc atc gcc caa gcc caa ccc gat acg gaa    1440
   Ile Ser Pro Lys Gly Asn Ile Ile Ala Gln Ala Gln Pro Asp Thr Glu
   465                 470                 475                 480
   acc gta ttg gaa gga cac atc aaa ggc tat gtc ggc gaa acg ccc tat    1488
   Thr Val Leu Glu Gly His Ile Lys Gly Tyr Val Gly Glu Thr Pro Tyr
                   485                 490                 495
   atg aaa acc ggc agt tca tgg tgg ttg atg ggc ata ttg acc cta gcc    1536
   Met Lys Thr Gly Ser Ser Trp Trp Leu Met Gly Ile Leu Thr Leu Ala
               500                 505                 510
   gca ctg att ctt ttc atc ttc cga aac aaa gaa cac                    1572
   Ala Leu Ile Leu Phe Ile Phe Arg Asn Lys Glu His
           515                 520
<210> 10
   <211> 524
   <212> PRT
   <213> Neisseria meningitidis
<400> 10
   Met Phe Arg Arg Tyr Leu Pro Asn Arg Gln Met Asn Met Val Ser Lys
    1               5                  10                  15
   Leu Asp Lys Tyr Trp Gln His Pro Ala Leu Tyr Trp Pro Leu Leu Ile
               20                  25                  30
   Leu Phe Ala Ala Ala Thr Pro Phe Thr Phe Ala Pro Tyr Tyr His Phe
           35                  40                  45
   Trp Leu Met Pro Leu Ile Phe Gly Ala Phe Val Arg Leu Ile Glu Leu
       50                  55                  60
   Arg Pro Arg Phe Ala Val Ser Ser Ala Tyr Leu Phe Gly Leu Thr Ala
   65                  70                  75                  80
   Tyr Thr Thr Gln Phe Tyr Trp Ile His Thr Ala Leu His Asp Val Ser
                   85                  90                  95
   Gly Leu Pro Asp Leu Tyr Ala Val Pro Leu Thr Phe Leu Leu Pro Ala
               100                 105                 110
   Tyr Leu Ala Leu Tyr Pro Ala Leu Cys Phe Trp Leu Trp Lys Lys Phe
               115                 120                 125
   Thr Leu Pro Arg Gly Ile Lys Ile Gly Leu Val Leu Pro Ile Leu Trp
       130                 135                 140
   Thr Leu Thr Glu Phe Ala Arg Glu Arg Phe Leu Thr Gly Phe Gly Trp
   145                 150                 155                 160
   Gly Ala Ile Gly Tyr Ser Gln Ile Thr Pro Asp Ser Pro Leu Ala Gly
                   165                 170                 175
   Phe Ala Pro Phe Gly Gly Ile His Met Val Thr Leu Ala Thr Ala Phe
               180                 185                 190
   Leu Gly Val Trp Leu Val Leu Ala Ser Asp Asn Thr Ala Arg Ser Gly
           195                 200                 205
   Lys Arg Leu Leu Pro Ile Ile Leu Ile Ala Ala Leu Leu Ala Ala Gly
       210                 215                 220
   Tyr Thr Ala Arg Gln Thr Asp Phe Thr Arg Pro Asp Gly Ser Arg Ser
   225                 230                 235                 240
   Thr Val Ala Leu Leu Gln Gly Asn Ile Asp Gln Thr Leu Lys Trp Arg
                   245                 250                 255
   Glu Asp Gln Val Ile Pro Thr Ile Gln Lys Tyr Tyr Glu Gln Val Gly
               260                 265                 270
   Lys Thr Thr Ala Asp Ile Val Ile Leu Pro Glu Thr Ala Ile Pro Val
           275                 280                 285
   Met Arg Gln Asn Leu Pro Glu Asn Ile Leu Ala Lys Phe Ala Glu Gln
       290                 295                 300
   Ala Gln Asn Asn Gly Ser Ala Leu Ala Val Gly Ile Ser Gln Tyr Thr
   305                 310                 315                 320
   Ser Asp Gly Asn Gly Tyr Glu Asn Ala Val Ile Asn Leu Thr Gly Tyr
                   325                 330                 335
   Gln Glu Asn Asn Gln Asp Gly Ile Pro Tyr Tyr Ala Lys Asn His Leu
               340                 345                 350
   Val Pro Phe Gly Glu Tyr Lys Pro Leu Pro Phe Leu Thr Thr Pro Leu
           355                 360                 365
   Tyr Lys Met Met Asn Met Pro Leu Ser Asp Phe Arg Lys Gly Gly Gly
           370                 375                 380
   Lys Gln Ser Ala Leu Leu Met Lys Asn Gln Lys Ile Ala Phe Asn Ile
   385                 390                 395                 400
   Cys Tyr Glu Asp Gly Phe Gly Asp Glu Leu Ile Ala Ala Ala Lys Asp
                   405                 410                 415
   Ala Thr Leu Leu Ala Asn Ala Ser Asn Met Ala Trp Tyr Gly Lys Ser
               420                 425                 430
   Asn Ala Met Tyr Gln His Leu Gln Gln Ser Gln Ala Arg Ala Met Glu
           435                 440                 445
   Leu Gly Arg Tyr Met Val Arg Ala Thr Asn Thr Gly Ala Thr Ala Ile
       450                 455                 460
   Ile Ser Pro Lys Gly Asn Ile Ile Ala Gln Ala Gln Pro Asp Thr Glu
   465                 470                 475                 480
   Thr Val Leu Glu Gly His Ile Lys Gly Tyr Val Gly Glu Thr Pro Tyr
                   485                 490                 495
   Met Lys Thr Gly Ser Ser Trp Trp Leu Met Gly Ile Leu Thr Leu Ala
               500                 505                 510
   Ala Leu Ile Leu Phe Ile Phe Arg Asn Lys Glu His
           515                 520
<210> 11
   <211> 1185
   <212> DNA
   <213> Neisseria meningitidis
<220>
   <221> CDS
   <222> (1)..(1185)
<400> 11
   atg aca gag gac gaa cgt ttc gcg tgg ctg caa ttg gcg ttt acg ccc    48
   Met Thr Glu Asp Glu Arg Phe Ala Trp Leu Gln Leu Ala Phe Thr Pro
     1               5                  10                  15
   tat atc ggc gcg gaa agt ttc ctg ctg ctg atg cgc cgt ttc ggc agc    96
   Tyr Ile Gly Ala Glu Ser Phe Leu Leu Leu Met Arg Arg Phe Gly Ser
                20                  25                  30
   gcg caa aat gcc ctg tcc gca ccg gcg gaa cag gtg gcg gca ctg ata     144
   Ala Gln Asn Ala Leu Ser Ala Pro Ala Glu Gln Val Ala Ala Leu Ile
               35                  40                  45
   cgg cac aaa cag gcg ctt gag gct tgg cgc aat gcg gaa aaa cgc gct     192
   Arg His Lys Gln Ala Leu Glu Ala Trp Arg Asn Ala Glu Lys Arg Ala
           50                  55                  60
   ctg gcg cgg cag gcg gca gaa gcg gcg ttg gaa tgg gaa atg cgg gac     240
   Leu Ala Arg Gln Ala Ala Glu Ala Ala Leu Glu Trp Glu Met Arg Asp
   65                  70                  75                  80
   gga tgc cgc ctg atg ctg ctt cag gat gaa gat ttc ccc gaa atg ctg     288
   Gly Cys Arg Leu Met Leu Leu Gln Asp Glu Asp Phe Pro Glu Met Leu
                   85                  90                  95
   acg cag ggg ctg acc gcg ccg ccg gtt ttg ttt ttg cgc ggc aac gtg     336
   Thr Gln Gly Leu Thr Ala Pro Pro Val Leu Phe Leu Arg Gly Asn Val
               100                 105                 110
   cgg ctg ctg cac aag cct tcc gcc gcc atc gtc ggc agc cgc cat gcc     384
   Arg Leu Leu His Lys Pro Ser Ala Ala Ile Val Gly Ser Arg His Ala
           115                 120                 125
   acg ccg cag gcg atg cgg att gcc aaa gat ttc ggc aag tcg ttg ggc     432
   Thr Pro Gln Ala Met Arg Ile Ala Lys Asp Phe Gly Lys Ser Leu Gly
       130                 135                 140
   ggg caa aat atc ccc gtt gtg tcg ggt atg gct tcc ggt atc gat act     480
   Gly Gln Asn Ile Pro Val Val Ser Gly Met Ala Ser Gly Ile Asp Thr
   145                 150                 155                 160
   gcc gcc cat cag ggc gca ctg gag gca gaa ggc ggc acc atc gcc gtg     528
   Ala Ala His Gln Gly Ala Leu Glu Ala Glu Gly Gly Thr Ile Ala Val
                   165                 170                 175
   tgg ggg acg ggt ata gac cgc att tac ccg ccg tcc aat aaa aac ctt     576
   Trp Gly Thr Gly Ile Asp Arg Ile Tyr Pro Pro Ser Asn Lys Asn Leu
               180                 185                 190
   gcc tat gaa atc gcc gaa aaa gga ttg att gtc agc gag ttt ccc atc     624
   Ala Tyr Glu Ile Ala Glu Lys Gly Leu Ile Val Ser Glu Phe Pro Ile
           195                 200                 205
   ggt acg cgc ccg tat gcc ggc aat ttt ccg cgc cgc aac cgc ctg att     672
   Gly Thr Arg Pro Tyr Ala Gly Asn Phe Pro Arg Arg Asn Arg Leu Ile
       210                 215                 220
   gcc gcc ctg tcg caa gta acg ctg gtg gtt gaa gcc gcc ttg gaa tcc     720
   Ala Ala Leu Ser Gln Val Thr Leu Val Val Glu Ala Ala Leu Glu Ser
   225                 230                 235                 240
   ggt tcg ctg att acc gcc ggc ctg gcg gca gaa atg ggg cgc gaa gtg     768
   Gly Ser Leu Ile Thr Ala Gly Leu Ala Ala Glu Met Gly Arg Glu Val
                   245                 250                 255
   atg gcg gta ccc ggt tcg ata gac aat ccg cac agc aaa ggc tgc cac     816
   Met Ala Val Pro Gly Ser Ile Asp Asn Pro His Ser Lys Gly Cys His
               260                 265                 270
   aaa ctg att aaa gac ggc gca aaa ttg gtg gaa tgc ctg gac gac atc     864
   Lys Leu Ile Lys Asp Gly Ala Lys Leu Val Glu Cys Leu Asp Asp Ile
           275                 280                 285
   ctg aac gaa tgc ccg ggg cta ttg caa aat acg ggt gct tca tca tat     912
   Leu Asn Glu Cys Pro Gly Leu Leu Gln Asn Thr Gly Ala Ser Ser Tyr
       290                 295                 300
   tct ata aat aag gat acg ccc gac acg gga cgc cgc gcc gtt cag acg     960
   Ser Ile Asn Lys Asp Thr Pro Asp Thr Gly Arg Arg Ala Val Gln Thr
   305                 310                 315                 320
   gca tac gcc ccg ccg cct gcc gca aaa atg ccg tct gaa gcg gcg gca     1008
   Ala Tyr Ala Pro Pro Pro Ala Ala Lys Met Pro Ser Glu Ala Ala Ala
                   325                 330                 335
   ggc ggc aca gcc gtc ggc ggc ata ctg gat aaa atg ggt ttc gac ccc     1056
   Gly Gly Thr Ala Val Gly Gly Ile Leu Asp Lys Met Gly Phe Asp Pro
               340                 345                 350
   atc cat ccc gac gtg ctt gcc gga cag ttg gct atg cct gcc gca gat     1104
   Ile His Pro Asp Val Leu Ala Gly Gln Leu Ala Met Pro Ala Ala Asp
           355                 360                 365
   ttg tat gcc gca ctg ttg gaa ttg gaa ttg gac ggc agc gtt gcc gca     1152
   Leu Tyr Ala Ala Leu Leu Glu Leu Glu Leu Asp Gly Ser Val Ala Ala
       370                 375                 380
   atg ccc agc ggc aga tac cag cgt atc cga act                         1185
   Met Pro Ser Gly Arg Tyr Gln Arg Ile Arg Thr
   385                 390                 395
<210> 12
   <211> 395
   <212> PRT
   <213> Neisseria meningitidis
<400> 12
   Met Thr Glu Asp Glu Arg Phe Ala Trp Leu Gln Leu Ala Phe Thr Pro
   1 5 10 15
   Tyr Ile Gly Ala Glu Ser Phe Leu Leu Leu Met Arg Arg Phe Gly Ser
   20 25 30
   Ala Gln Asn Ala Leu Ser Ala Pro Ala Glu Gln Val Ala Ala Leu Ile
   35 40 45
   Arg His Lys Gln Ala Leu Glu Ala Trp Arg Asn Ala Glu Lys Arg Ala
   50 55 60
   Leu Ala Arg Gln Ala Ala Glu Ala Ala Leu Glu Trp Glu Met Arg Asp
   65 70 75 80
   Gly Cys Arg Leu Met Leu Leu Gln Asp Glu Asp Phe Pro Glu Met Leu
   85 90 95
   Thr Gln Gly Leu Thr Ala Pro Pro Val Leu Phe Leu Arg Gly Asn Val
   100 105 110
   Arg Leu Leu His Lys Pro Ser Ala Ala Ile Val Gly Ser Arg His Ala
   115 120 125
   Thr Pro Gln Ala Met Arg Ile Ala Lys Asp Phe Gly Lys Ser Leu Gly
   130 135 140
   Gly Gln Asn Ile Pro Val Val Ser Gly Met Ala Ser Gly Ile Asp Thr
   145 150 155 160
   Ala Ala His Gln Gly Ala Leu Glu Ala Glu Gly Gly Thr Ile Ala Val
   165 170 175
   Trp Gly Thr Gly Ile Asp Arg Ile Tyr Pro Pro Ser Asn Lys Asn Leu
   180 185 190
   Ala Tyr Glu Ile Ala Glu Lys Gly Leu Ile Val Ser Glu Phe Pro Ile
   195 200 205
   Gly Thr Arg Pro Tyr Ala Gly Asn Phe Pro Arg Arg Asn Arg Leu Ile
   210 215 220
   Ala Ala Leu Ser Gln Val Thr Leu Val Val Glu Ala Ala Leu Glu Ser
   225 230 235 240
   Gly Ser Leu Ile Thr Ala Gly Leu Ala Ala Glu Met Gly Arg Glu Val
                   245                 250                 255
   Met Ala Val Pro Gly Ser Ile Asp Asn Pro His Ser Lys Gly Cys His
               260                 265                 270
   Lys Leu Ile Lys Asp Gly Ala Lys Leu Val Glu Cys Leu Asp Asp Ile
           275                 280                 285
   Leu Asn Glu Cys Pro Gly Leu Leu Gln Asn Thr Gly Ala Ser Ser Tyr
       290                 295                 300
   Ser Ile Asn Lys Asp Thr Pro Asp Thr Gly Arg Arg Ala Val Gln Thr
   305                 310                 315                 320
   Ala Tyr Ala Pro Pro Pro Ala Ala Lys Met Pro Ser Glu Ala Ala Ala
                   325                 330                 335
   Gly Gly Thr Ala Val Gly Gly Ile Leu Asp Lys Met Gly Phe Asp Pro
               340                 345                 350
   Ile His Pro Asp Val Leu Ala Gly Gln Leu Ala Met Pro Ala Ala Asp
           355                 360                 365
   Leu Tyr Ala Ala Leu Leu Glu Leu Glu Leu Asp Gly Ser Val Ala Ala
       370                 375                 380
   Met Pro Ser Gly Arg Tyr Gln Arg Ile Arg Thr
   385                 390                 395
<210> 13
   <211> 804
   <212> DNA
   <213> Neisseria meningitidis
<220>
   <221> CDS
   <222> (1)..(804)
<400> 13
   atg ccg aca gtt ttc atc ttt ttg acg gcg gtt tac gca gga ttg ggt     48
   Met Pro Thr Val Phe Ile Phe Leu Thr Ala Val Tyr Ala Gly Leu Gly
   1               5                   10                  15
   gca ttt gca tgg cac tgc caa cag cag ggg ctc ggc cgg gat tac ccg     96
   Ala Phe Ala Trp His Cys Gln Gln Gln Gly Leu Gly Arg Asp Tyr Pro
               20                  25                  30
   tgg aag acg gaa ttg ccg gtt ttg ggc gcg gca ttg acc gtc cat ggc    144
   Trp Lys Thr Glu Leu Pro Val Leu Gly Ala Ala Leu Thr Val His Gly
           35                  40                  45
   gcg gca ctg ctt atg ccg gtc att caa gac aaa atc atc att atg ggc    192
   Ala Ala Leu Leu Met Pro Val Ile Gln Asp Lys Ile Ile Ile Met Gly
       50                  55                  60
   ttt ggg tat tcc ggc agc ctg att gtt tgg atg atg ctg ttt att tat    240
   Phe Gly Tyr Ser Gly Ser Leu Ile Val Trp Met Met Leu Phe Ile Tyr
   65                  70                  75                  80
   ttt gcc ggc agc ttc ttt tat tcg ctg cgc gga gtg cag ttg ctg ctg    288
   Phe Ala Gly Ser Phe Phe Tyr Ser Leu Arg Gly Val Gln Leu Leu Leu
                   85                  90                  95
   tat cct tgc gcc gca ctg atg ctg ctg tca ggt ttg gtt ttt cct gga    336
   Tyr Pro Cys Ala Ala Leu Met Leu Leu Ser Gly Leu Val Phe Pro Gly
               100                 105                 110
   aaa ttc tcg gga tat gaa att acc gac ctt ccc ttt atg ctg cat atc    384
   Lys Phe Ser Gly Tyr Glu Ile Thr Asp Leu Pro Phe Met Leu His Ile
           115                 120                 125
   gga act tcg ctg ctt gca tac ggg ctg ttc ggc atc gca aca tta ttg    432
   Gly Thr Ser Leu Leu Ala Tyr Gly Leu Phe Gly Ile Ala Thr Leu Leu
       130                 135                 140
   tcc gtt ttg acc ctg ctg ctg aat cgg agc ctg cac cgc agg aac ttc    480
   Ser Val Leu Thr Leu Leu Leu Asn Arg Ser Leu His Arg Arg Asn Phe
   145                 150                 155                 160
   tcc aag ctc gca gga ttc ctg ccg tcg ctg ctc agt ttg gaa aaa ctc    528
   Ser Lys Leu Ala Gly Phe Leu Pro Ser Leu Leu Ser Leu Glu Lys Leu
                   165                 170                 175
   atg ttc cag gcc atg tgg gca ggt ttc atc ctg ctg acc tat tcc gtc    576
   Met Phe Gln Ala Met Trp Ala Gly Phe Ile Leu Leu Thr Tyr Ser Val
               180                 185                 190
   gtc agc gga aca ttt ttt gcc gaa gcc gta ttc ggc aaa ccc atg acc    624
   Val Ser Gly Thr Phe Phe Ala Glu Ala Val Phe Gly Lys Pro Met Thr
           195                 200                 205
   ttt acc cat aaa acc gta ttc ggc ata ttg tca tgg ctg att tac ggc    672
   Phe Thr His Lys Thr Val Phe Gly Ile Leu Ser Trp Leu Ile Tyr Gly
       210                 215                 220
   gga ctg ctg ctc aag cac agc atg acc gca tgg cgc ggc aaa aaa gcc    720
   Gly Leu Leu Leu Lys His Ser Met Thr Ala Trp Arg Gly Lys Lys Ala
   225                 230                 235                 240
   gcc gtg tgg acc atc atc gga ttt gtc agc ctg atg att gcc tat atg    768
   Ala Val Trp Thr Ile Ile Gly Phe Val Ser Leu Met Ile Ala Tyr Met
                   245                 250                 255
   ggc agc aag ttc gta ttg gaa atc att ctg aac aga                    804
   Gly Ser Lys Phe Val Leu Glu Ile Ile Leu Asn Arg
               260                 265
<210> 14
   <211> 268
   <212> PRT
   <213> Neisseria meningitidis
<400> 14
   Met Pro Thr Val Phe Ile Phe Leu Thr Ala Val Tyr Ala Gly Leu Gly
    1               5                  10                  15
   Ala Phe Ala Trp His Cys Gln Gln Gln Gly Leu Gly Arg Asp Tyr Pro
               20                  25                  30
   Trp Lys Thr Glu Leu Pro Val Leu Gly Ala Ala Leu Thr Val His Gly
           35                  40                  45
   Ala Ala Leu Leu Met Pro Val Ile Gln Asp Lys Ile Ile Ile Met Gly
       50                  55                  60
   Phe Gly Tyr Ser Gly Ser Leu Ile Val Trp Met Met Leu Phe Ile Tyr
   65                  70                  75                  80
   Phe Ala Gly Ser Phe Phe Tyr Ser Leu Arg Gly Val Gln Leu Leu Leu
                   85                  90                  95
   Tyr Pro Cys Ala Ala Leu Met Leu Leu Ser Gly Leu Val Phe Pro Gly
               100                 105                 110
   Lys Phe Ser Gly Tyr Glu Ile Thr Asp Leu Pro Phe Met Leu His Ile
           115                 120                 125
   Gly Thr Ser Leu Leu Ala Tyr Gly Leu Phe Gly Ile Ala Thr Leu Leu
       130                 135                 140
   Ser Val Leu Thr Leu Leu Leu Asn Arg Ser Leu His Arg Arg Asn Phe
   145                 150                 155                 160
   Ser Lys Leu Ala Gly Phe Leu Pro Ser Leu Leu Ser Leu Glu Lys Leu
                   165                 170                 175
   Met Phe Gln Ala Met Trp Ala Gly Phe Ile Leu Leu Thr Tyr Ser Val
               180                 185                 190
   Val Ser Gly Thr Phe Phe Ala Glu Ala Val Phe Gly Lys Pro Met Thr
           195                 200                 205
   Phe Thr His Lys Thr Val Phe Gly Ile Leu Ser Trp Leu Ile Tyr Gly
       210                 215                 220
   Gly Leu Leu Leu Lys His Ser Met Thr Ala Trp Arg Gly Lys Lys Ala
   225                 230                 235                 240
   Ala Val Trp Thr Ile Ile Gly Phe Val Ser Leu Met Ile Ala Tyr Met
                   245                 250                 255
   Gly Ser Lys Phe Val Leu Glu Ile Ile Leu Asn Arg
               260                 265
<210> 15
   <211> 2391
   <212> DNA
   <213> Neisseria meningitidis
<220>
   <221> CDS
   <222> (1)..(2391)
<400> 15
   atg tca aca gtc caa atg aat aaa aat att aaa tct tta aat tta cgg     48
   Met Ser Thr Val Gln Met Asn Lys Asn Ile Lys Ser Leu Asn Leu Arg
     1               5                  10                  15
   gaa aaa gac ccg ttt tta agt cgt gaa aaa cag cgt tat gaa cat cct     96
   Glu Lys Asp Pro Phe Leu Ser Arg Glu Lys Gln Arg Tyr Glu His Pro
                20                  25                  30
   ttg ccc agt cgg gaa tgg ata atc gag ctg ctt gaa cgt aaa ggc gta    144
   Leu Pro Ser Arg Glu Trp Ile Ile Glu Leu Leu Glu Arg Lys Gly Val
            35                  40                  45
   cca tcc aag att gaa gct ttg gta cgc gaa ttg tcg att aag gaa gaa    192
   Pro Ser Lys Ile Glu Ala Leu Val Arg Glu Leu Ser Ile Lys Glu Glu
        50                  55                  60
   gag tac gaa ttt ttc gaa cgt cgt ctg aag gcg atg gcg cgg gac ggt    240
   Glu Tyr Glu Phe Phe Glu Arg Arg Leu Lys Ala Met Ala Arg Asp Gly
    65                  70                  75                  80
   cag gtt tta atc aac cgt cgg ggc gcg gtt tgc gcg gcg gac aaa ttg    288
   Gln Val Leu Ile Asn Arg Arg Gly Ala Val Cys Ala Ala Asp Lys Leu
                    85                  90                  95
   gat ttg gtc aaa tgc cgt gtc gag gcg cac aaa gac ggc ttc ggt ttc    336
   Asp Leu Val Lys Cys Arg Val Glu Ala His Lys Asp Gly Phe Gly Phe
               100                 105                 110
   gcc gtg ccg ctc acg ccc gcc aaa gac ggt gat ttt gtt ttg tac gaa    384
   Ala Val Pro Leu Thr Pro Ala Lys Asp Gly Asp Phe Val Leu Tyr Glu
           115                 120                 125
   cgc cag atg cgc ggc att atg cac ggc gat att gtc act gtt cgt cct    432
   Arg Gln Met Arg Gly Ile Met His Gly Asp Ile Val Thr Val Arg Pro
       130                 135                 140
   gcc ggc atg gac cgt agg ggc cgc cgc gaa ggg acg gtt ctg gat att    480
   Ala Gly Met Asp Arg Arg Gly Arg Arg Glu Gly Thr Val Leu Asp Ile
   145                 150                 155                 160
   gtc gaa cgc gcg caa agc aaa gtg gtc ggc cgt ttc tat atg gat agg    528
   Val Glu Arg Ala Gln Ser Lys Val Val Gly Arg Phe Tyr Met Asp Arg
                   165                 170                 175
   ggc gtg gcg att ttg gag ccg gaa gac aag cgt ctg aac caa agc atc    576
   Gly Val Ala Ile Leu Glu Pro Glu Asp Lys Arg Leu Asn Gln Ser Ile
               180                 185                 190
   gta ttg gaa ccg gac ggc gtg gcg cgt ttc aaa cct gaa tcc ggt cag    624
   Val Leu Glu Pro Asp Gly Val Ala Arg Phe Lys Pro Glu Ser Gly Gln
           195                 200                 205
   gtc atc gtc ggc gaa att gag gtt tat cct gag caa aac cgg ccg gca    672
   Val Ile Val Gly Glu Ile Glu Val Tyr Pro Glu Gln Asn Arg Pro Ala
       210                 215                 220
   gtg gca aaa atc atc gaa gtt ttg ggc gat tat gcc gac agc ggc atg    720
   Val Ala Lys Ile Ile Glu Val Leu Gly Asp Tyr Ala Asp Ser Gly Met
   225                 230                 235                 240
   gag att gaa att gcc gtg cgc aag cat cat ttg ccg cac caa ttc agt    768
   Glu Ile Glu Ile Ala Val Arg Lys His His Leu Pro His Gln Phe Ser
                   245                 250                 255
   gaa gcg tgt gcc aaa gcc gcg aaa aaa att ccc gac cat gta cgc aaa    816
   Glu Ala Cys Ala Lys Ala Ala Lys Lys Ile Pro Asp His Val Arg Lys
               260                 265                 270
   agc gat ttg aaa ggc cgc gtc gat ttg cgc gac ctg cct ttg gta acg    864
   Ser Asp Leu Lys Gly Arg Val Asp Leu Arg Asp Leu Pro Leu Val Thr
           275                 280                 285
   ata gac ggc gaa acg gct cga gat ttt gac gat gcg gtg ttt gcc gag    912
   Ile Asp Gly Glu Thr Ala Arg Asp Phe Asp Asp Ala Val Phe Ala Glu
       290                 295                 300
   aaa atc gga cgc aat tac cgt ctg gtc gtg gcg att gcc gat gtc agc    960
   Lys Ile Gly Arg Asn Tyr Arg Leu Val Val Ala Ile Ala Asp Val Ser
   305                 310                 315                 320
   cat tat gtc cgc ccc gat gac gct atc gac acg gac gct cag gaa cgc    1008
   His Tyr Val Arg Pro Asp Asp Ala Ile Asp Thr Asp Ala Gln Glu Arg
                   325                 330                 335
   agc acc agt gtt tac ttc ccg cgc cgc gtg att ccc atg ttg ccg gaa    1056
   Ser Thr Ser Val Tyr Phe Pro Arg Arg Val Ile Pro Met Leu Pro Glu
               340                 345                 350
   aac ctg tcc aac ggc atc tgc tcg ctc aat cct cat gtc gag cgt ttg    1104
   Asn Leu Ser Asn Gly Ile Cys Ser Leu Asn Pro His Val Glu Arg Leu
           355                 360                 365
   tgt gtg gtg tgc gat atg gtt atc act tac gcg ggc aat atc aaa gaa    1152
   Cys Val Val Cys Asp Met Val Ile Thr Tyr Ala Gly Asn Ile Lys Glu
       370                 375                 380
   tac cgc ttc tac ccc gcc gtg atg cgc tct cat gcc cgc ctg acc tac    1200
   Tyr Arg Phe Tyr Pro Ala Val Met Arg Ser His Ala Arg Leu Thr Tyr
   385                 390                 395                 400
   aac caa gtt tgg aaa tgg ctt tca ggc ggc atc gag cat ccg ttc aaa    1248
   Asn Gln Val Trp Lys Trp Leu Ser Gly Gly Ile Glu His Pro Phe Lys
                   405                 410                 415
   acc caa atc gac acg ctt tac aaa ctc ttc aaa atc ctt cag aaa aag    1296
   Thr Gln Ile Asp Thr Leu Tyr Lys Leu Phe Lys Ile Leu Gln Lys Lys
               420                 425                 430
   cgt ttc gaa cgc ggg gcg gtg gag ttt gac agc atc gaa acc caa atg      1344
   Arg Phe Glu Arg Gly Ala Val Glu Phe Asp Ser Ile Glu Thr Gln Met
           435                 440                 445
   ctt ttc gac gac aac ggt aaa att gaa aaa atc gtc ccc gtt gtc cgc      1392
   Leu Phe Asp Asp Asn Gly Lys Ile Glu Lys Ile Val Pro Val Val Arg
       450                 455                 460
   aac gat gcc cac aag ctg att gaa gaa tgt atg ttg gcg gca aac gtt      1440
   Asn Asp Ala His Lys Leu Ile Glu Glu Cys Met Leu Ala Ala Asn Val
   465                 470                 475                 480
   tgc gca gcg gat ttt ctg ttg aaa aac aag cat acc gca ttg ttc cgc      1488
   Cys Ala Ala Asp Phe Leu Leu Lys Asn Lys His Thr Ala Leu Phe Arg
                   485                 490                 495
   aac cat ttg ggg ccc acg ccc gaa aaa ctc gcc gcc ttg cgc gag cag      1536
   Asn His Leu Gly Pro Thr Pro Glu Lys Leu Ala Ala Leu Arg Glu Gln
               500                 505                 510
   ctc ggt ctg ttg ggg ctt caa ctt ggc ggc ggc gac aac ccg tcg ccg      1584
   Leu Gly Leu Leu Gly Leu Gln Leu Gly Gly Gly Asp Asn Pro Ser Pro
           515                 520                 525
   aaa gac tat gcc gcg ctt gcc gga cag ttc aaa ggc agg ccg gat gcc      1632
   Lys Asp Tyr Ala Ala Leu Ala Gly Gln Phe Lys Gly Arg Pro Asp Ala
       530                 535                 540
   gaa ttg ctg caa gtc atg atg ttg cgc tcc atg caa cag gcg gtt tac      1680
   Glu Leu Leu Gln Val Met Met Leu Arg Ser Met Gln Gln Ala Val Tyr
   545                 550                 555                 560
   gaa ccg cat tgc gac gga cac ttt ggt ctt gcc tac gaa gca tac gcc      1728
   Glu Pro His Cys Asp Gly His Phe Gly Leu Ala Tyr Glu Ala Tyr Ala
                   565                 570                 575
   cac ttc acc tcg ccc atc cgc cgc tat ccc gac ctg acc gta cac cgc      1776
   His Phe Thr Ser Pro Ile Arg Arg Tyr Pro Asp Leu Thr Val His Arg
               580                 585                 590
   gcc atc aaa gcc gtg ttg aat cag caa acc tac acg cca aaa aaa agc      1824
   Ala Ile Lys Ala Val Leu Asn Gln Gln Thr Tyr Thr Pro Lys Lys Ser
           595                 600                 605
   tgg cag gct ttg ggc gtg cat acc tcg ttc tgt gag cgc cgt gcc gac      1872
   Trp Gln Ala Leu Gly Val His Thr Ser Phe Cys Glu Arg Arg Ala Asp
       610                 615                 620
   gac gcc agc cgc gac gtg gaa aac tgg ctg aaa acc tat tat atg cgc     1920
   Asp Ala Ser Arg Asp Val Glu Asn Trp Leu Lys Thr Tyr Tyr Met Arg
   625                 630                 635                 640
   gat aag gtc ggc gaa gta ttc gaa ggt aaa atc tcc ggc atg acc agt     1968
   Asp Lys Val Gly Glu Val Phe Glu Gly Lys Ile Ser Gly Met Thr Ser
                   645                 650                 655
   ttt ggt atc ttt gta aca ctg gac ggc atc cac att gac ggc ttg gtg     2016
   Phe Gly Ile Phe Val Thr Leu Asp Gly Ile His Ile Asp Gly Leu Val
               660                 665                 670
   cat atc agc gat ttg ggc gaa gac tat ttc aac ttc cgc ccc gaa atc     2064
   His Ile Ser Asp Leu Gly Glu Asp Tyr Phe Asn Phe Arg Pro Glu Ile
           675                 680                 685
   atg gca atc gaa ggc gaa cgc agc ggc atc cgt ttc aac atg ggg gac     2112
   Met Ala Ile Glu Gly Glu Arg Ser Gly Ile Arg Phe Asn Met Gly Asp
       690                 695                 700
   agg gtt gcc gtc cgg gtc gcc cgt gcc gat ttg gat gac gga aaa atc     2160
   Arg Val Ala Val Arg Val Ala Arg Ala Asp Leu Asp Asp Gly Lys Ile
   705                 710                 715                 720
   gat ttt gtc ctg att gcc ggg ggg agc ggc agg ggg cgg aaa gtt aaa     2208
   Asp Phe Val Leu Ile Ala Gly Gly Ser Gly Arg Gly Arg Lys Val Lys
                   725                 730                 735
   tca tcc gcg tct gcc aaa ccg gca ggg acg gcg ggg aaa ggg aag ccg     2256
   Ser Ser Ala Ser Ala Lys Pro Ala Gly Thr Ala Gly Lys Gly Lys Pro
               740                 745                 750
   aaa acc gcc gcc gag aaa aaa aca gcc cga ggc ggc aaa gta agg gga     2304
   Lys Thr Ala Ala Glu Lys Lys Thr Ala Arg Gly Gly Lys Val Arg Gly
           755                 760                 765
   agg ggc gcg tct gcc gcc gca gaa tcg agg aaa aag gca aag aaa ccg     2352
   Arg Gly Ala Ser Ala Ala Ala Glu Ser Arg Lys Lys Ala Lys Lys Pro
       770                 775                 780
   gtt ccg att aag gta aaa aaa cgg aaa ggc aaa tca taa                 2391
   Val Pro Ile Lys Val Lys Lys Arg Lys Gly Lys Ser
   785                 790                 795
<210> 16
   <211> 796
   <212> PRT
   <213> Neisseria meningitidis
<400> 16
   Met Ser Thr Val Gln Met Asn Lys Asn Ile Lys Ser Leu Asn Leu Arg
   1 5 10 15
   Glu Lys Asp Pro Phe Leu Ser Arg Glu Lys Gln Arg Tyr Glu His Pro
   20 25 30
   Leu Pro Ser Arg Glu Trp Ile Ile Glu Leu Leu Glu Arg Lys Gly Val
   35 40 45
   Pro Ser Lys Ile Glu Ala Leu Val Arg Glu Leu Ser Ile Lys Glu Glu
   50 55 60
   Glu Tyr Glu Phe Phe Glu Arg Arg Leu Lys Ala Met Ala Arg Asp Gly
   65 70 75 80
   Gln Val Leu Ile Asn Arg Arg Gly Ala Val Cys Ala Ala Asp Lys Leu
   85 90 95
   Asp Leu Val Lys Cys Arg Val Glu Ala His Lys Asp Gly Phe Gly Phe
   100 105 110
   Ala Val Pro Leu Thr Pro Ala Lys Asp Gly Asp Phe Val Leu Tyr Glu
   115 120 125
   Arg Gln Met Arg Gly Ile Met His Gly Asp Ile Val Thr Val Arg Pro
   130 135 140
   Ala Gly Met Asp Arg Arg Gly Arg Arg Glu Gly Thr Val Leu Asp Ile
   145 150 155 160
   Val Glu Arg Ala Gln Ser Lys Val Val Gly Arg Phe Tyr Met Asp Arg
   165 170 175
   Gly Val Ala Ile Leu Glu Pro Glu Asp Lys Arg Leu Asn Gln Ser Ile
   180 185 190
   Val Leu Glu Pro Asp Gly Val Ala Arg Phe Lys Pro Glu Ser Gly Gln
   195 200 205
   Val Ile Val Gly Glu Ile Glu Val Tyr Pro Glu Gln Asn Arg Pro Ala
   210 215 220
   Val Ala Lys Ile Ile Glu Val Leu Gly Asp Tyr Ala Asp Ser Gly Met
   225 230 235 240
   Glu Ile Glu Ile Ala Val Arg Lys His His Leu Pro His Gln Phe Ser
                   245                 250                 255
   Glu Ala Cys Ala Lys Ala Ala Lys Lys Ile Pro Asp His Val Arg Lys
               260                 265                 270
   Ser Asp Leu Lys Gly Arg Val Asp Leu Arg Asp Leu Pro Leu Val Thr
           275                 280                 285
   Ile Asp Gly Glu Thr Ala Arg Asp Phe Asp Asp Ala Val Phe Ala Glu
       290                 295                 300
   Lys Ile Gly Arg Asn Tyr Arg Leu Val Val Ala Ile Ala Asp Val Ser
   305                 310                 315                 320
   His Tyr Val Arg Pro Asp Asp Ala Ile Asp Thr Asp Ala Gln Glu Arg
                   325                 330                 335
   Ser Thr Ser Val Tyr Phe Pro Arg Arg Val Ile Pro Met Leu Pro Glu
               340                 345                 350
   Asn Leu Ser Asn Gly Ile Cys Ser Leu Asn Pro His Val Glu Arg Leu
           355                 360                 365
   Cys Val Val Cys Asp Met Val Ile Thr Tyr Ala Gly Asn Ile Lys Glu
       370                 375                 380
   Tyr Arg Phe Tyr Pro Ala Val Met Arg Ser His Ala Arg Leu Thr Tyr
   385                 390                 395                 400
   Asn Gln Val Trp Lys Trp Leu Ser Gly Gly Ile Glu His Pro Phe Lys
                   405                 410                 415
   Thr Gln Ile Asp Thr Leu Tyr Lys Leu Phe Lys Ile Leu Gln Lys Lys
               420                 425                 430
   Arg Phe Glu Arg Gly Ala Val Glu Phe Asp Ser Ile Glu Thr Gln Met
           435                 440                 445
   Leu Phe Asp Asp Asn Gly Lys Ile Glu Lys Ile Val Pro Val Val Arg
       450                 455                 460
   Asn Asp Ala His Lys Leu Ile Glu Glu Cys Met Leu Ala Ala Asn Val
   465                 470                 475                 480
   Cys Ala Ala Asp Phe Leu Leu Lys Asn Lys His Thr Ala Leu Phe Arg
                   485                 490                 495
   Asn His Leu Gly Pro Thr Pro Glu Lys Leu Ala Ala Leu Arg Glu Gln
   500 505 510
   Leu Gly Leu Leu Gly Leu Gln Leu Gly Gly Gly Asp Asn Pro Ser Pro
   515 520 525
   Lys Asp Tyr Ala Ala Leu Ala Gly Gln Phe Lys Gly Arg Pro Asp Ala
   530 535 540
   Glu Leu Leu Gln Val Met Met Leu Arg Ser Met Gln Gln Ala Val Tyr
   545 550 555 560
   Glu Pro His Cys Asp Gly His Phe Gly Leu Ala Tyr Glu Ala Tyr Ala
   565 570 575
   His Phe Thr Ser Pro Ile Arg Arg Tyr Pro Asp Leu Thr Val His Arg
   580 585 590
   Ala Ile Lys Ala Val Leu Asn Gln Gln Thr Tyr Thr Pro Lys Lys Ser
   595 600 605
   Trp Gln Ala Leu Gly Val His Thr Ser Phe Cys Glu Arg Arg Ala Asp
   610 615 620
   Asp Ala Ser Arg Asp Val Glu Asn Trp Leu Lys Thr Tyr Tyr Met Arg
   625 630 635 640
   Asp Lys Val Gly Glu Val Phe Glu Gly Lys Ile Ser Gly Met Thr Ser
   645 650 655
   Phe Gly Ile Phe Val Thr Leu Asp Gly Ile His Ile Asp Gly Leu Val
   660 665 670
   His Ile Ser Asp Leu Gly Glu Asp Tyr Phe Asn Phe Arg Pro Glu Ile
   675 680 685
   Met Ala Ile Glu Gly Glu Arg Ser Gly Ile Arg Phe Asn Met Gly Asp
   690 695 700
   Arg Val Ala Val Arg Val Ala Arg Ala Asp Leu Asp Asp Gly Lys Ile
   705 710 715 720
   Asp Phe Val Leu Ile Ala Gly Gly Ser Gly Arg Gly Arg Lys Val Lys
   725 730 735
   Ser Ser Ala Ser Ala Lys Pro Ala Gly Thr Ala Gly Lys Gly Lys Pro
   740 745 750
   Lys Thr Ala Ala Glu Lys Lys Thr Ala Arg Gly Gly Lys Val Arg Gly
   755 760 765
   Arg Gly Ala Ser Ala Ala Ala Glu Ser Arg Lys Lys Ala Lys Lys Pro
   770 775 780
   Val Pro Ile Lys Val Lys Lys Arg Lys Gly Lys Ser
   785 790 795
<210> 17
   <211> 252
   <212> DNA
   <213> Neisseria meningitidis
<220>
   <221> CDS
   <222> (1)..(252)
<400> 17
   atg gag cag tcg ggc aaa ttc agt tgg tct gcg gca gct ttt tgg gac 48
   Met Glu Gln Ser Gly Lys Phe Ser Trp Ser Ala Ala Ala Phe Trp Asp
   1 5 10 15
   att ccc tac ccc gcc acc agg cgg att gcc tca agt ttg tat tcg acc 96
   Ile Pro Tyr Pro Ala Thr Arg Arg Ile Ala Ser Ser Leu Tyr Ser Thr
   20 25 30
   gaa tat ttt gtc gta tgc ttt cta cgt ttg atg cca ctc tct ccg tgt 144
   Glu Tyr Phe Val Val Cys Phe Leu Arg Leu Met Pro Leu Ser Pro Cys
   35 40 45
   aat ctg tat ttt gtc acc cat ctg cgt acc aat gaa tcg gaa ata gaa 192
   Asn Leu Tyr Phe Val Thr His Leu Arg Thr Asn Glu Ser Glu Ile Glu
   50 55 60
   aga tgg tct gct gtt ccc tgc caa ata gta ttg aac gac ggc aag tcg 240
   Arg Trp Ser Ala Val Pro Cys Gln Ile Val Leu Asn Asp Gly Lys Ser
   65 70 75 80
   gaa ttc atc tga 252
   Glu Phe Ile
<210> 18
   <211> 83
<212> PRT
   <213> Neisseria meningitidis
<400> 18
   Met Glu Gln Ser Gly Lys Phe Ser Trp Ser Ala Ala Ala Phe Trp Asp
   1               5                   10                  15
   Ile Pro Tyr Pro Ala Thr Arg Arg Ile Ala Ser Ser Leu Tyr Ser Thr
               20                  25                  30
   Glu Tyr Phe Val Val Cys Phe Leu Arg Leu Met Pro Leu Ser Pro Cys
           35                  40                  45
   Asn Leu Tyr Phe Val Thr His Leu Arg Thr Asn Glu Ser Glu Ile Glu
       50                  55                  60
   Arg Trp Ser Ala Val Pro Cys Gln Ile Val Leu Asn Asp Gly Lys Ser
   65                  70                  75                  80
   Glu Phe Ile
<210> 19
   <211> 789
   <212> DNA
   <213> Neisseria meningitidis
<220>
   <221> CDS
   <222> (1)..(789)
<400> 19
   atg aat gtt tat ttt ctc ggc ggc ggc aat atg gcg gct gcc gtt gcg    48
   Met Asn Val Tyr Phe Leu Gly Gly Gly Asn Met Ala Ala Ala Val Ala
    1               5                  10                  15
   ggc gga ttg gtc aaa caa ggc ggt tac cgc atc cat ata gcc aat cgg    96
   Gly Gly Leu Val Lys Gln Gly Gly Tyr Arg Ile His Ile Ala Asn Arg
               20                  25                  30
   ggt gcg gaa aaa cgc gaa cgt ttg gga aaa gag ttg ggg gtc gaa act   144
   Gly Ala Glu Lys Arg Glu Arg Leu Gly Lys Glu Leu Gly Val Glu Thr
           35                  40                  45
   tcg gca acc ctg ccg gag ctt cat tcc gac gat gtt tta atc ctt gcc   192
   Ser Ala Thr Leu Pro Glu Leu His Ser Asp Asp Val Leu Ile Leu Ala
       50                  55                  60
   gtc aaa ccg cag gat atg gaa gcc gcg tgc aaa aat atc cgc acc aat    240
   Val Lys Pro Gln Asp Met Glu Ala Ala Cys Lys Asn Ile Arg Thr Asn
    65                 70                  75                  80
   ggt gca ttg gtg ctt tct gtc gca gct gga ttg tcg gtt gat acg ctc    288
   Gly Ala Leu Val Leu Ser Val Ala Ala Gly Leu Ser Val Asp Thr Leu
                   85                  90                  95
   agc cgt tac ctc ggg gga aca cgc cgc att gtc cgg gtt atg ccg aat    336
   Ser Arg Tyr Leu Gly Gly Thr Arg Arg Ile Val Arg Val Met Pro Asn
               100                 105                 110
   aca ccc gga aaa atc ggg ctg ggc gta tcc ggt atg ttt gcc gaa gcg    384
   Thr Pro Gly Lys Ile Gly Leu Gly Val Ser Gly Met Phe Ala Glu Ala
           115                 120                 125
   gaa gta tcg gaa aca gac cgc agg att gcc gat cga atc atg aaa tca    432
   Glu Val Ser Glu Thr Asp Arg Arg Ile Ala Asp Arg Ile Met Lys Ser
       130                 135                 140
   gtc ggt ttg acc gtt tgg ttg gaa gat gaa gcg caa atg cac agc att    480
   Val Gly Leu Thr Val Trp Leu Glu Asp Glu Ala Gln Met His Ser Ile
   145                 150                 155                 160
   acc ggt atc agc ggc agc ggg ccg gca tat gtg ttt tat ctg ctg gac    528
   Thr Gly Ile Ser Gly Ser Gly Pro Ala Tyr Val Phe Tyr Leu Leu Asp
                   165                 170                 175
   gca ttg caa aat gcc gcc atc cga caa ggg ttt gat atg gca gaa gca    576
   Ala Leu Gln Asn Ala Ala Ile Arg Gln Gly Phe Asp Met Ala Glu Ala
               180                 185                 190
   cgc gcg ctc agt ctg gca acg ttt aaa gga gcg gtt gcc ctt gcc gag    624
   Arg Ala Leu Ser Leu Ala Thr Phe Lys Gly Ala Val Ala Leu Ala Glu
           195                 200                 205
   cag acg ggt gaa gat ttc gag aag ctt caa aaa aat gta acg tca aaa    672
   Gln Thr Gly Glu Asp Phe Glu Lys Leu Gln Lys Asn Val Thr Ser Lys
       210                 215                 220
   ggc ggg aca acc cac gaa gcc gtg gaa gct ttc agg cgg cat cgt gtc    720
   Gly Gly Thr Thr His Glu Ala Val Glu Ala Phe Arg Arg His Arg Val
   225                 230                 235                 240
   gcc gaa gcc ata agc gag ggc gtt tgt gcc tgt gtg cgc cgt tcg cag    768
   Ala Glu Ala Ile Ser Glu Gly Val Cys Ala Cys Val Arg Arg Ser Gln
                   245                 250                 255
   gaa atg gaa cgg caa tat caa                                  789
   Glu Met Glu Arg Gln Tyr Gln
                   260
<210> 20
   <211> 263
   <212> PRT
   <213> Neisseria meningitidis
<400> 20
   Met Asn Val Tyr Phe Leu Gly Gly Gly Asn Met Ala Ala Ala Val Ala
    1               5                  10                  15
   Gly Gly Leu Val Lys Gln Gly Gly Tyr Arg Ile His Ile Ala Asn Arg
               20                  25                  30
   Gly Ala Glu Lys Arg Glu Arg Leu Gly Lys Glu Leu Gly Val Glu Thr
           35                  40                  45
   Ser Ala Thr Leu Pro Glu Leu His Ser Asp Asp Val Leu Ile Leu Ala
       50                  55                  60
   Val Lys Pro Gln Asp Met Glu Ala Ala Cys Lys Asn Ile Arg Thr Asn
   65                  70                  75                  80
   Gly Ala Leu Val Leu Ser Val Ala Ala Gly Leu Ser Val Asp Thr Leu
                   85                  90                  95
   Ser Arg Tyr Leu Gly Gly Thr Arg Arg Ile Val Arg Val Met Pro Asn
               100                 105                 110
   Thr Pro Gly Lys Ile Gly Leu Gly Val Ser Gly Met Phe Ala Glu Ala
           115                 120                 125
   Glu Val Ser Glu Thr Asp Arg Arg Ile Ala Asp Arg Ile Met Lys Ser
       130                 135                 140
   Val Gly Leu Thr Val Trp Leu Glu Asp Glu Ala Gln Met His Ser Ile
   145                 150                 155                 160
   Thr Gly Ile Ser Gly Ser Gly Pro Ala Tyr Val Phe Tyr Leu Leu Asp
                   165                 170                 175
   Ala Leu Gln Asn Ala Ala Ile Arg Gln Gly Phe Asp Met Ala Glu Ala
               180                 185                 190
   Arg Ala Leu Ser Leu Ala Thr Phe Lys Gly Ala Val Ala Leu Ala Glu
           195                 200                 205
   Gln Thr Gly Glu Asp Phe Glu Lys Leu Gln Lys Asn Val Thr Ser Lys
       210                 215                 220
   Gly Gly Thr Thr His Glu Ala Val Glu Ala Phe Arg Arg His Arg Val
   225                 230                 235                 240
   Ala Glu Ala Ile Ser Glu Gly Val Cys Ala Cys Val Arg Arg Ser Gln
                   245                 250                 255
   Glu Met Glu Arg Gln Tyr Gln
               260
<210> 21
   <211> 132
   <212> DNA
   <213> Neisseria meningitidis
<220>
   <221> CDS
   <222> (1) .. (132)
<400> 21
   atg ggg aaa cgg ctg atg ccg tct gaa ggg aaa ggt gcg gca ggc aat    48
   Met Gly Lys Arg Leu Met Pro Ser Glu Gly Lys Gly Ala Ala Gly Asn
     1               5                  10                  15
   ccg gag gcg cat aaa aaa gca gat ata ttc gga ctg cac ctc ccg aat    96
   Pro Glu Ala His Lys Lys Ala Asp Ile Phe Gly Leu His Leu Pro Asn
                20                  25                  30
   ata tct gcc tgc tgt ttc ctc ttt att cag cct tta                    132
   Ile Ser Ala Cys Cys Phe Leu Phe Ile Gln Pro Leu
            35                  40
<210> 22
   <211> 44
   <212> PRT
   <213> Neisseria meningitidis
<400> 22
   Met Gly Lys Arg Leu Met Pro Ser Glu Gly Lys Gly Ala Ala Gly Asn
    1               5                  10                  15
   Pro Glu Ala His Lys Lys Ala Asp Ile Phe Gly Leu His Leu Pro Asn
                20                  25                  30
   Ile Ser Ala Cys Cys Phe Leu Phe Ile Gln Pro Leu
            35                  40